# FASCICULE DE BREVET EUROPEEN

(11) **EP 0 637 334 B1**
(45) Date de publication et mention de la délivrance du brevet: **06.06.2007**
(21) Numéro de dépôt: 93911529.1
(22) Date de dépôt: 19.04.1993
(51) Int. Cl.: C12N 15/12, C12P 21/08, A61K 39/395, G01N 33/577, C12Q 1/68

(54) **PEPTIDES AYANT UNE ACTIVITE DE FACTEUR D'ECHANGE DU GDP, SEQUENCES D'ACIDES NUCLEIQUES CODANT POUR CES PEPTIDES, PREPARATION ET UTILISATION**
PEPTIDE MIT GDP-AUSTAUSCHFAKTORARTIGER WIRKUNG, DAFÜR KODIERENDE NUKLEINSÄURESEQUENZEN, IHRE HERSTELLUNG UND VERWENDUNG
PEPTIDES HAVING A GDP EXCHANGE FACTOR ACTIVITY, NUCLEIC ACID SEQUENCES CODING FOR SAID PEPTIDES, PREPARATION AND UTILIZATION

(30) Priorité: 21.04.1992 FR 9204827
(43) Date de publication de la demande: 08.02.1995
(73) Titulaire: Aventis Pharma S.A., 92160 Antony (FR)
(72) Inventeur: SCHWEIGHOFFER, Fabien, F-94300 Vincennes (FR); TOCQUE, Bruno, 92400 COURBEVOIE (FR)
(74) Mandataire: Becker, Philippe
(86) Numéro de dépôt international: PCT/FR1993/000382
(87) Numéro de publication internationale: WO 1993/021314

(56) Documents cités:
- ONCOGENE vol. 5, no. 9, Septembre 1990, pages 1321 - 1328 YASUO FUKUMOTO ET AL. 'Molecular cloning and characterization of a novel type of regulatory protein (GDI) for the rho proteins, ras p21-like small GTP-binding proteins'
- MOLECULAR AND CELLULAR BIOLOGY vol. 10, no. 8, Août 1990, WASHINGTON US pages 4116 - 4122 YASUSHI MATSUI ET AL. 'Molecular cloning and characterization of a novel type of regulatory protein (GDI) for smg p25A, a ras p21-like GTP-binding protein'
- JOURNAL OF CELLULAR BIOCHEMISTRY vol. 16B, 8 Février 1992, page 220 RENATA ZIPPEL ET AL. 'CDC25 proteins in growth regulation and signal transduction in yeast and mammalian cells' cité dans la demande
- DATABASE PUBMED [en ligne] NCBI 1994 CHARDIN; MATTEI Database accession no. PMID: 8275713

## Description

La présente invention concerne de nouvelles séquences peptidiques et nucléotidiques, et leur utilisation pharmaceutique. Plus particulièrement, l'invention concerne des peptides capables de moduler les niveaux d'échange du GDP sur des complexes p21-GDP.

Les produits des gènes ras, généralement désignés protéines p21, jouent un rôle clé dans le contrôle de la division cellulaire chez tous les organismes eucaryotes où ils ont été recherchés. Certaines modifications spécifiques de ces protéines leur font perdre leur contrôle normal et les conduisent à devenir oncogéniques. Ainsi, un grand nombre de tumeurs humaines ont été associées à la présence de gènes ras modifiés. De même, une surexpression de ces protéines p21 peut conduire à un dérèglement de la prolifération cellulaire. La compréhension du rôle exact de ces protéines p21 dans les cellules, de leur mode de fonctionnement et de leurs caractéristiques constitue donc un enjeu majeur pour la compréhension et l'approche thérapeutique de la cancérogénèse.

*In vivo,* on ne connait pas encore la nature exacte des événements responsables de l'activation des protéines p21. On sait qu'elles exercent leurs fonctions en oscillant entre deux états conformationnels : une forme inactive liée au GDP et une forme active liée au GTP, mais les facteurs agissant sur la transition entre ces deux formes ne sont pas clairement identifiés. Des travaux récents rapportent des situations physiologiques au cours desquelles la proportion de protéines ras liées au GTP augmente dans la cellule. Il s'agit de l'activation des lymphocytes T et de la stimulation des fibroblastes 3T3 par des facteurs de croissance dont l'EGF et le PDGF (Downward et al., Nature 346 (1990) 719 ; Gibbs et al., J. Biol. Chem. 265 (1990) 20437). L'augmentation de la proportion de p21-GTP peut s'expliquer au moins en partie par l'action d'une protéine jouant un rôle analogue à celui d'un récepteur pour les protéines G de transduction. A cet égard, certaines protéines capables de promouvoir l'échange du GDP sur les protéines p21 ont été identifiées, à partir de cerveau de boeuf [West et coll., FEBS Lett. 259 (1990) 245] et de rat [Wolfman et Macara, Science 248 (1990) 67]. La localisation cellulaire distincte de ces facteurs et les conditions expérimentales très différentes dans lesquelles ils ont été obtenus laissent supposer qu'il s'agit de protéines différentes. Elles sont aussi actives sur les protéines ras normales que sur celles qui sont oncogéniques. Ces activités sont regroupées sous le terme de GEF : Facteur d'Echange des nucléotides Guanidiques, ou GRF.

Chez la levure *Saccharomyces cerevisiae,* l'activité GRF a été attribuée au produit du gène CDC25 [Camonis et al., EMBO J. 5 (1986) 375], et des études ont été réalisées afin de comprendre la voie de signalisation faisant intervenir le produit des gènes CDC25, RAS1 et RAS2 d'une part et l'adénylate cyclase d'autre part chez la levure *Saccharomyces cerevisiae.* En particulier, de nombreux travaux ont été focalisés sur la caractérisation du produit du gène CDC25 qui était l'élément le plus mal connu de cette chaîne. Le produit du gène CDC25 constitue l'élément le plus en amont de la cascade de réactions conduisant à l'activation de la p21 chez la levure. Les travaux réalisés dans ce domaine ont contribué à démontrer que le produit de ce gène devait agir comme facteur d'échange GDP -> GTP pour activer les protéines ras. Un second gène de la levure *S. cerevisiae,* SDC25, structurellement très voisin de CDC25, a été isolé et caractérisé. Le domaine actif de SDC25 semble être un facteur d'échange capable d'agir *in vitro* et *in vivo* sur les protéines ras. Ce domaine constitue le premier constituant moléculaire décrit doué de cette activité.

Très récemment, une protéine de type GRF a été également mise en évidence chez la souris [Vanoni et Martegani, J. Cell. Bioch. Suppl.16B (1992) 220]. La purification d'une protéine d'origine bovine, appelée *rho* GDI (GDP Dissociation Inhibitor), qui régule l'échange GDP-GTP de la protéine rho a été décrite (Fukumoto et al. Oncogene (1990), 5, pp. 1321), ainsi que que le clonage d'une protéine d'origine bovine, appelée *smg* p25A GDI (GDP Dissociation Inhibitor), qui régule l'échange GDP-GTP de la protéine *smg* p25A (Matsui et *al.* Molecular and Cellular Biology (Aug. 1990) p. 4116-4122)

Toutefois, jusqu'à aujourd'hui, aucune activité GRF n'a été isolée et caractérisée chez l'homme. La présente invention résulte précisément de la démonstration par la demanderesse de l'existence d'un facteur humain d'échange du GDP. La présente invention résulte plus particulièrement de l'identification, de l'isolement et de la caractérisation de peptides et de séquences nucléotidiques d'origine humaine, désignés hGRF et hSOS, capables de moduler l'état d'activation des protéines p21.

Un premier aspect de l'invention consiste donc en des peptides utilisables pharmaceutiquement. Plus particulièrement, un objet de l'invention réside dans des peptides capables de moduler les niveaux d'échange du GDP sur des complexes p21-GDP. Il est entendu que p21 désigne tout produit d'expression d'un gène ras normal ou oncogénique.

Plus particulièrement, les peptides de l'invention sont choisis parmi tout ou partie des séquences SEQ ID n° 2, 3, 4, 6 ou 8 ou d'un dérivé de celles-ci.

Au sens de la présente invention, le terme dérivé désigne toute molécule obtenue par modification de nature génétique et/ou chimique de ces séquences et conservant l'activité recherchée. Par modification de nature génétique et/ou chimique, on doit entendre toute mutation, substitution, délétion, addition et/ou modification d'un ou plusieurs résidus. De tels dérivés peuvent être générés dans des buts différents, tels que notamment celui d'augmenter l'affinité du peptide pour son site d'interaction, celui d'améliorer ses niveaux de production, celui d'augmenter sa résistance à des protéases, celui d'augmenter son efficacité thérapeutique ou de réduire ses effets secondaires, ou celui de lui conférer de nouvelles propriétés pharmacocinétiques et/ou biologiques.

Dans un mode particulier de l'invention, les peptides de l'invention sont des peptides capables de stimuler l'échange du GDP sur le complexe p21-GDP.

Dans un autre mode particulier de l'invention, les peptides de l'invention sont des peptides capables de ralentir ou d'inhiber l'échange du GDP sur le complexe p21-GDP. De tels peptides sont préférentiellement des peptides capables d'antagoniser l'interaction du facteur d'échange du GDP avec le complexe p21-GDP. Il peut donc s'agir de fragments des séquences indiquées ci-dessus ou de dérivés de celles-ci. De tels fragments peuvent être générés de différentes façons. En particulier, ils peuvent être synthétisés par voie chimique, sur la base des séquences données dans la présente demande, en utilisant les synthétiseurs peptidiques connus de l'homme du métier. Ils peuvent également être synthétisés par voie génétique, par expression dans un hôte cellulaire d'une séquence nucléotidique codant pour le peptide recherché. Dans ce cas, la séquence nucléotidique peut être préparée chimiquement en utilisant un synthétiseur d'oligonucléotides, sur la base de la séquence peptidique donnée dans la présente demande et du code génétique. La séquence nucléotidique peut également être préparée à partir des séquences données dans la présente demande (SEQ ID n° 1, 5 et 7), par coupures enzymatiques, ligature, clonage, etc, selon les techniques connues de l'homme du métier, ou par criblage de banques d'ADN avec des sondes élaborées à partir de ces séquences. Par ailleurs, les peptides de l'invention capables de ralentir ou d'inhiber l'échange du GDP sur le complexe p21-GDP peuvent également être des peptides ayant une séquence correspondant au site d'interaction du facteur d'échange sur le complexe p21-GDP.

Un autre objet de l'invention réside dans des anticorps ou fragments d'anticorps polyclonaux ou monoclonaux dirigés contre un peptide tel que défini ci-avant. De tels anticorps peuvent être générés par des méthodes connues de l'homme du métier, compte tenu des enseignements donnés dans la présente demande. En particulier, ces anticorps peuvent être préparés par immunisation d'un animal contre un peptide de l'invention, prélèvement du sang, et isolement des anticorps. Ces anticorps peuvent également être générés par préparation d'hybridomes selon les techniques connues de l'homme de l'art.

Plus préférentiellement, les anticorps ou fragments d'anticorps de l'invention présentent la capacité d'inhiber au moins partiellement l'interaction du facteur d'échange avec le complexe p21-GDP. Ils peuvent ainsi être utilisés pour réguler l'etat d'activation du produit des gènes ras.

Par ailleurs, ces anticorps peuvent également être utilisés pour détecter et/ou doser le facteur d'échange du GDP humain dans des échantillons biologiques, et de ce fait, pour renseigner sur l'état d'activation du produit des gènes ras.

La présente invention permet donc de générer des peptides dérivés des séquences SEQ ID n° 2-4, 6 et 8, ainsi que des anticorps dirigés contre ces peptides, présentant des propriétés biologiques interessantes en vue d'une utilisation pharmaceutique. L'activité biologique des différents peptides et anticorps de l'invention sur l'échange du GDP peut être évaluée de différentes façon ainsi qu'illustré dans les exemples.

L'invention fournit également des composés non peptidiques ou non exclusivement peptidiques utilisables pharmaceutiquement. Il est en effet possible, à partir des motifs protéiques actifs décrits dans la présente demande, de réaliser des molécules inhibitrices de la voie de signalisation dépendante des protéines ras non exclusivement peptidiques et compatibles avec une utilisation pharmaceutique. A cet égard, l'invention concerne l'utilisation d'un polypeptide de l'invention tel que décrit ci-avant pour la préparation de molécules non-peptidiques, ou non exclusivement peptidiques, actives pharmacologiquement sur les niveaux d'échange du GDP, par détermination des éléments structuraux de ce polypeptide qui sont importants pour son activité et reproduction de ces éléments par des structures non-peptidiques ou non exclusivement peptidiques. L'invention a aussi pour objet des compositions pharmaceutiques comprenant une ou plusieurs molécules ainsi préparées.

La présente invention a également pour objet toute séquence d'acide nucléique codant pour un polypeptide tel que défini ci-dessus. Plus préférentiellement, il s'agit d'une séquence choisie parmi :
(a) tout ou partie des séquences SEQ ID n° 1, 5 ou 7 ou de leur brin complémentaire,
(b) toute séquence hybridant avec une séquence (a) et codant pour un polypeptide selon l'invention, et
(c) les séquences dérivées des séquences (a) et (b) en raison de la dégénérescence du code génétique.

Les différentes séquences nucléotidiques de l'invention peuvent être d'origine artificielle ou non. Il peut s'agir de séquences génomiques, d'ADNc, d'ARN, de séquences hybrides ou de séquences synthétiques ou semi-synthétiques. Ces séquences peuvent être obtenues par exemple par criblage de banques d'ADN (banque d'ADNc, banque d'ADN génomique) au moyen de sondes élaborées sur la base des séquences SEQ ID n° 1, 5 ou 7. De telles banques peuvent être préparées à partir de cellules de différentes origines par des techniques classiques de biologie moléculaires connues de l'homme du métier. Les séquences nucléotidiques de l'invention peuvent également être préparées par synthèse chimique, notamment selon la méthode des phosphoramidites, ou encore par des méthodes mixtes incluant la modification chimique ou enzymatique de séquences obtenues par criblage de banques.

Ces séquences nucléotidiques selon l'invention sont utilisables dans le domaine pharmaceutique, soit pour la production in vitro des peptides de l'invention, soit pour la réalisation de séquences antisens ou pour la production des peptides de l'invention dans le cadre d'une thérapie génique, soit encore pour la détection et le diagnostic, par des expériences d'hybri-dation, de l'expression ou d'une surexpression d'un facteur d'échange du GDP amplifié, muté ou réarrangé dans des échantillons biologiques ou pour l'isolement de séquences homologues à partir d'autres sources cellulaires.

Pour la production des peptides de l'invention, les séquences nucléiques définies ci-dessus sont généralement placées sous le contrôle de signaux permettant leur expression dans un hôte cellulaire. Le choix de ces signaux (promoteurs, terminateurs, séquence "leader" de sécrétion, etc) peut varier en fonction de l'hôte cellulaire utilisé. Préférentiellement, ces séquences nucléotidiques de l'invention font partie d'un vecteur, qui peut être à réplication autonome ou intégratif. Plus particulièrement, des vecteurs à réplication autonome peuvent être préparés en utilisant des séquences à réplication autonome chez l'hôte choisi. S'agissant des vecteurs intégratifs, ceux-ci peuvent être préparés par exemple en utilisant des séquences homologues à certaines régions du génome de l'hôte, permettant, par recombinaison homologue, l'intégration du vecteur.

Les hôtes cellulaires utilisables pour la production des peptides de l'invention sont aussi bien des hôtes eucaryotes que procaryotes. Parmi les hôtes eucaryotes qui conviennent, on peut citer les cellules animales, les levures, ou les champignons. En particulier, s'agissant de levures, on peut citer les levures du genre *Saccharomyces, Kluyveromyces, Pichia, Schwanniomyces,* ou *Hansenula.* S'agissant de cellules animales, on peut citer les cellules COS, CHO, C127, etc. Parmi les champignons, on peut citer plus particulièrement *Aspergillus* ssp. ou *Trichoderma* ssp. Comme hôtes procaryotes, on préfère utiliser les bactéries suivantes *E.coli, Bacillus,* ou *Streptomyces.*

Les séquences d'acides nucléiques selon l'invention peuvent également servir à la réalisation d'oligonucléotides antisens ou d'antisens génétiques utilisables comme agents pharmaceutiques. L'inhibition de l'expression de certains oncogènes par des séquences antisens s'est avérée être une stratégie utile dans la compréhension du rôle de ces oncogènes et une voie particulièrement prometteuse dans la réalisation d'un traitement anticancéreux. Les séquences antisens sont des oliogonucléotides de petite taille, complémentaire du brin codant d'un gène donné, et de ce fait capables d'hybrider spécifiquement avec l'ARNm transcrit, inhibant sa traduction en protéine. L'invention a ainsi pour objet les séquences antisens capables d'inhiber au moins partiellement la production de peptides stimulant l'échange du GDP sur des complexes p21-GDP. De telles séquences peuvent être constituées par tout ou partie des séquences nucléiques définies ci-avant. Il s'agit généralement de séquences ou de fragments de séquences complémentaires de séquences codant pour des peptides stimulant l'échange du GDP. De tels oligonucléotides peuvent être obtenus à partir des séquences SEQ ID n° 1, 5 ou 7, par fragmentation, etc, ou par synthèse chimique. De telles séquences peuvent être utilisées dans le cadre de thérapies géniques, pour le transfert et l'expression in vivo de séquences antisens ou de peptides capables de moduler les niveaux d'échanges du GDP sur les protéines ras. A cet égard, les séquences peuvent être incorporées dans des vecteurs, notamment d'origine virale.

L'invention concerne également, comme séquences nucléotidiques, les sondes nucléotidiques, synthétiques ou non, capables de s'hydrider avec les séquences nucléotidiques définies ci-avant qui codent pour un peptide de l'invention, ou avec l'ARNm correspondant. De telles sondes peuvent être utilisées in vitro comme outil de diagnostic, pour la détection de l'expression du facteur d'échange du GDP, ou encore pour la mise en évidence d'anomalies génétiques (mauvais épissage, polymorphisme, mutations ponctuelles, etc). De telles sondes doivent être préalablement marquées, et pour cela différentes techniques sont connues de l'homme du métier. Les conditions d'hybridation dans lesquelles ces sondes peuvent être utilisées sont les conditions normales de stringence (voir notamment les techniques générales de clonage ci-après ainsi que les exemples). Ces sondes peuvent également être utilisées pour la mise en évidence et l'isolement de séquences d'acides nucléiques homologues codant pour un peptide de l'invention, à partir d'autres sources cellulaires, ainsi qu'illustré dans les exemples.

L'invention a encore pour objet toute composition pharmaceutique comprenant comme principe actif au moins un peptide tel que défini ci-avant.

Elle a aussi pour objet toute composition pharmaceutique comprenant comme principe actif au moins un anticorps et/ou un fragment d'anticorps tel que défini ci-avant, ainsi que toute composition pharmaceutique comprenant comme principe actif au moins une séquence nucléotidique telle que définie ci-avant.

Par ailleurs, elle a aussi pour objet les compositions pharmaceutiques dans lesquelles les peptides, anticorps et séquence nucléotidique définis ci-avant sont associés entre-eux ou avec d'autres principes actifs.

Les compositions pharmaceutiques selon l'invention peuvent être utilisées pour moduler l'activation des protéines p21 et de ce fait pour moduler la prolifération de certains types cellulaires. Plus particulièrement, ces compositions pharmaceutiques sont destinées au traitement de cancers. De nombreux cancers ont en effet été associés à la présence de protéines ras oncogéniques. Parmi les cancers renfermant le plus souvent des gènes ras mutés, on peut citer notamment les adénocarcinomes du pancréas, dont 90 % ont un oncogène Ki-ras muté sur le douzième codon [Almoguera et coll., Cell 53 (1988) 549], les adénocarcinomes du colon et les cancers de la thyroïde (50 %), ou les carcinomes du poumon et les leucémies myéloïdes [30 %. Bos, J.L. Cancer Res. 49 (1989) 4682].

L'invention a encore pour objet l'utilisation des molécules décrites ci-avant pour moduler l'activité des protéines p21. En particulier, l'invention concerne l'utilisation de ces molécules pour inhiber au moins partiellement l'activation des protéines p21.

L'invention fournit également un procédé de détection de l'expression et/ou d'une surexpression d'un gène ras dans un échantillon biologique. Un tel procédé comprend par exemple la mise en contact d'un tel échantillon avec un anticorps ou fragment d'anticorps selon l'invention, la révélation des complexes antigène-anticorps, et la comparaison des résultats obtenus avec un échantillon standard. Dans un tel procédé, l'anticorps peut être en suspension ou préalablement immobilisé sur un support. Ce procédé peut également comprendre la mise en contact de l'échantillon avec une sonde nucléotidique selon l'invention, la mise en évidence des hybrides obtenus, et la comparaison avec ceux obtenus dans le cas d'un échantillon standard.

La présente invention peut être utilisée dans le domaine thérapeutique : les peptides, anticorps et séquences nucléotidiques de l'invention étant capables de moduler l'activité des gènes ras, ils permettent en effet d'intervenir dans le processus de développement des cancers. Ainsi qu'illustré dans les exemples, les séquences nucléotidiques de l'invention permettent notamment d'exprimer des peptides capables de complémenter la thermosensibilité de levures portant une mutation cdc25. Elles permettent également d'exprimer des peptides capables de supprimer une mutation dominante RAS2ts, démontrant une compétition avec le produit normal d'expression du gène CDC25 pour l'interaction avec les protéines p21. L'invention peut également être utilisée dans le domaine du diagnostic et du typage de cancers : les anticorps et sondes nucléotidiques de l'invention permettent en effet l'identification des cancers dans lesquels un gène ras est impliqué ainsi que le diagnostic de cancers liés à la surexpression d'un gène ras normal ou oncogénique.

D'autres avantages de la présente invention apparaîtront à la lecture des exemples qui suivent, qui doivent être considérés comme illustratifs et non limitatifs.

### Légende des figures

Figure 1 : Test de transactivation : Cette figure présente en ordonnée les niveaux d'activité CAT (% par rapport au bruit de fond) des souches CHO recombinantes, en fonction de la séquence d'ADNc utilisée pour la transformation.
Figure 2 : Test d'activité d'échange du GTP : Cette figure présente en ordonnée le rapport des formes p21-GDP/p21-GTP des souches CHO recombinantes, en fonction de la séquence d'ADNc utilisée pour la transformation.
Figure 3 : Test d'activité d'échange du GDP *in vitro* : Cette figure montre, en fonction du temps et pour 2 concentrations d'un peptide de l'invention, la diminution de la proportion de GDP rstant lié à la protéine p21.

### Techniques générales de clonage

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extractions de protéines au phénol ou au phénol-chloroforme, la précipitation d'ADN en milieu salin par de l'éthanol ou de l'isopropanol, la transformation dans *Escherichia coli,* etc, sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982; Ausubel F.M. et al. (eds), "Current Protocols in Molecular Biology", John Wiley & Sons, New York, 1987].

Les enzymes de restriction ont été fournies par New England Biolabs (Biolabs), Bethesda Research Laboratories (BRL) ou Amersham et sont utilisées selon les recommandations des fournisseurs.

Les plasmides de type pBR322, pUC, λgt11, pGEX 2T et les phages de la série M13 sont d'origine commerciale.

Pour les ligatures, les fragments d'ADN sont séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Biolabs) selon les recommandations du fournisseur.

Le remplissage des extrémités 5' proéminentes est effectué par le fragment de Klenow de l'ADN Polymérase I d*'E. coli* (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.

La mutagénèse dirigée *in vitro* par oligodéoxynucléotides synthétiques est effectuée selon la méthode développée par Taylor et al. [Nucleic Acids Res. 13 (1985) 8749-8764] en utilisant le kit distribué par Amersham.

L'amplification enzymatique de fragments d'ADN par la technique dite de PCR [Polymérase-catalyzed Chain Reaction, Saiki R.K. et al., Science 230 (1985) 1350-1354 ; Mullis K.B. et Faloona F.A., Meth. Enzym. 155 (1987) 335-350] est effectuée en utilisant un "DNA thermal cycler" (Perkin Elmer Cetus) selon les spécifications du fabricant.

La vérification des séquences nucléotidiques est effectuée par la méthode développée par Sanger et al. [Proc. Natl. Acad. Sci. USA, 74 (1977) 5463-5467] en utilisant le kit distribué par Amersham.

Pour les expériences d'hybridation, les conditions de stringence normales sont généralement les suivantes : hybridation : 3 x SCC en présence de 5 x Denhart's à 65°C ; lavage: 0,5 x SSC à 65°C.

### 1. Isolement du gène du facteur humain d'échange du GDP (hGRF)

5.10⁵ phages d'une banque de cerveau humain construite dans le vecteur λgt11 [Skolnik et al., Cell 65 (1991) 83] ont été criblés selon les techniques décrites par Sambrook, Fritsch et Maniatis (Molecular cloning ; Cold Spring Harbor Laboratory Press ; 1989).

La sonde utilisée pour le criblage de cette banque est un fragment d'ADNc humain de 137 paires de bases marqué au ³²P. Cette sonde a été préparée par PCR sur l'ADN total de la banque précitée en utilisant comme amorces les oligonucléotides dégénérés suivants : pour l'oligonucléotide de l'extrémité 3' du fragment (SEQ ID n° 11), et pour l'oligonucléotide de l'extrémité 5' du fragment (SEQ ID n° 12).

La sonde a été marquée au ³²P par "random priming" selon la technique de Feinberg et Vogelstein [Anal. Biochem. 137 (1984) 266], et la réaction de PCR a été menée à 40°C environ dans les conditions décrites dans les techniques générales de clonage.

Parmi les différents clones positifs obtenus par hybridation avec cette sonde, un comprend la totalité d'une phase ouverte qui porte l'activité d'échange vis-à-vis de Ha-Ras.

Ce clone de λgt11 contient un ADNc de 3 kb qui a été introduit, sous forme d'un fragment EcoRI, au site correspondant d'un vecteur M13mp18. Cet ADNc a ensuite été séquencé à l'aide des amorces commerciales "reverse" et "-20" ainsi qu'à l'aide d'oligonucléotides spécifiques selon la technique de Sanger (Cf techniques générales de clonage).

La séquence nucléotidique du gène du facteur humain d'échange du GDP porté par le fragment ainsi obtenu est présentée sur la séquence SEQ ID n° 1, ainsi que les séquences peptidiques déduites (SEQ ID n°2, 3 et 4).

### 2. Préparation de sous fragments

Différents dérivés ou fragments du gène ainsi obtenu peuvent être préparés et utilisés, notamment pour l'expression de peptides de l'invention. En particulier, les fragments suivants ont été préparés par coupure enzymatique, et séparés par électroélution :
- un fragment PstI-EcoRI comprenant une partie de la région codante du facteur d'échange (du nucléotide 936 jusqu'au codon stop) ainsi que la région 3' non codante du fragment,
- un fragment EcoNI-EcoRI comprenant une partie de la région codante du facteur d'échange (du nucléotide 910 jusqu'au codon stop) ainsi que la région 3' non codante du fragment,
- un fragment EagI-EcoRI comprenant une partie de la région codante du facteur d'échange (du nucléotide 638 jusqu'au codon stop) ainsi que la région 3' non codante du fragment,
- un fragment BaII-EcoRI comprenant une partie de la région codante du facteur d'échange (du nucléotide 88 jusqu'au codon stop) ainsi que la région 3' non codante du fragment, et,
- un fragment NaeI-EcoRI comprenant une partie de la région codante du facteur d'échange (du nucléotide 196 jusqu'au codon stop) ainsi que la région 3' non codante du fragment,

Il est entendu que la région 3' non codante portée par ces fragments est accessoire et qu'elle peut être éliminée, soit par digestion au moyen d'une nucléase, soit par coupure avec une enzyme ayant un site proche du codon stop, telle que notamment SmaI, dont le site est localisé environ 30 pb après le codon stop.

Il est entendu également que d'autres fragments peuvent être préparés, tels que notamment des fragments ne contenant pas la région codant pour la partie C-terminale entière, ainsi que des dérivés de ces fragments, obtenus par mutation, substitution, addition, ou modification de nature chimique et/ou génétique.

### 3. Caractérisation biologique

Les fonctionnalités des peptides selon l'invention ont été testées :
- dans des cellules mammifères,
- dans la levure *Saccharomyces cerevisiae,* ou encore
- *in vitro* sur la protéine Ha-Ras recombinante.

3.1. Pour l'évaluation fonctionnelle dans les cellules mammifères, les séquences d'ADN codant pour des peptides de l'invention, tels que par exemple ceux décrits dans l'exemple 2, peuvent être placées sous contrôle du promoteur précoce de SV40 dans le vecteur pCym1 décrit par Camonis et al. [Gene 86 (1990) 263].

Dans cet exemple, les fragments PstI-EcoRI et EcoNI-EcoRI décrit dans l'exemple 2 ont été insérés dans ce vecteur.

Les vecteurs ainsi obtenus ont été testés par transfection transitoire dans des cellules CHO selon le protocole décrit par Rey et al. [Oncogene 6 (1991) 347].

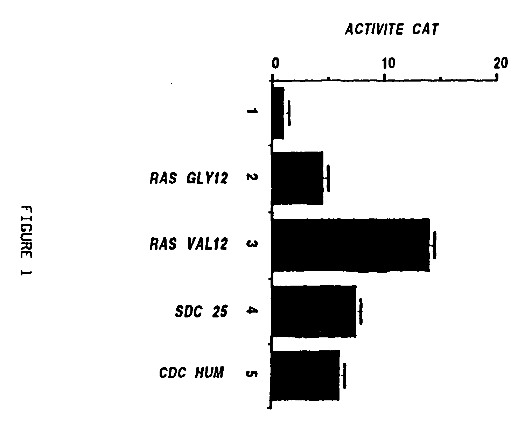

Deux critères de fonctionnalité ont ainsi été étudiés :
- la capacité des vecteurs à transactiver un promoteur gouvernant l'expression d'un gène reporteur qui est ici le gène bactérien codant pour la chloramphénicol acétyl transférase (gène CAT),
- leur capacité à promouvoir la charge en GTP des protéines Ras des cellules CHO transfectées.
   a) Pour les tests de transactivation, des cellules CHO à 50 % de confluence ont été transfectées (voir par exemple le protocole décrit par Schweighoffer et al. Science, In Press) d'une part avec 0,5 µg d'un vecteur portant le gène CAT sous contrôle d'un promoteur synthétique composé du promoteur murin du gène de la thymidine kinase et de 4 éléments PEA1 répétés dérivés de l'enhancer du polyôme [Wasylyk et al., EMBO J. 7 (1988) 2475], et d'autre part avec 4,5 µg d'un vecteur d'expression portant, sous contrôle du promoteur précoce de SV40, aucun ADNc codant (piste 1), l'ADNc de Ha-Ras normal (piste 2), l'ADNc de Ha-Ras activé en 12 (Val 12) (piste 3), l'extrémité 3' du cDNA de SDC25 décrite par Rey et al. précitée (piste 4), et l'ADNc codant pour un peptide selon l'invention décrit ci-dessus (piste 5).
      Les résultats sont présentés sur la figure 1. La piste 1 correspondant à l'activation basale, la piste 3 (obtenue pour le fragment PstI-EcoRI : CDC hum.) montre que l'expression d'un peptide de l'invention permet la transactivation du promoteur synthétique utilisé.
      Le même résultat qualitatif a été obtenu pour les autres fragments étudiés.
   b) De façon à vérifier que cette transactivation implique bien une charge nucléotidique des protéines ras des cellules CHO, les mêmes transfections transitoires ont été réalisées, le milieu de culture étant additionné d'orthophosphate marqué au ³²P.

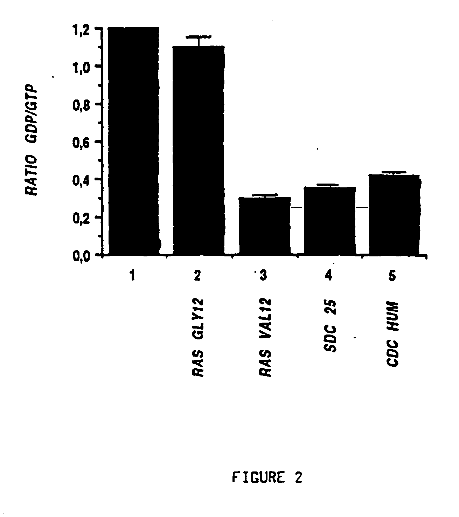

Ce protocole de marquage ainsi que celui de l'immunoprécipitation des protéines ras cellulaires est décrit par Rey et al. précitée. Les résultats obtenus sont présentés sur la figure 2. Ils montrent que les peptides de l'invention sont capables de moduler les niveaux d'échange du GDP sur les protéines ras puisque certains d'entre-eux (peptide CDC Hum. exprimé par le fragment PstI-EcoRI décrit ci-avant) sont capables de promouvoir la charge en GTP des protéines ras de cellules CHO immunoprécipitées par l'anticorps Y 13-259.

3.2. Les peptides de l'invention ont également été testés fonctionnellement dans la levure *S.cerevisiae* cdc25⁻. Pour cela, les vecteurs décrits précédemment (3.1.), qui sont des vecteurs navettes, ont été introduits dans la souche de levure OL97-1-11B [Camonis et Jacquet, Mol. Cell. Biol. 8 (1988) 2980]. Les résultats obtenus montrent que les fragments d'ADNc selon l'invention codent pour des peptides qui sont capables de complémenter le défaut de croissance de cette souche à 36°C. Ces résultats montrent ainsi que ces fragments codent pour des peptides fonctionnels in vivo dans *S.cerevisiae.*

3.3. La capacité des séquences d'ADN de l'invention à coder pour des peptides capables de promouvoir l'échange du GDP sur des protéines Ha-Ras purifiées a également été démontrée *in vitro* selon le protocole décrit par Rey et al. Mol. Cell. Biol. 9 (1989) 3904].

Pour cela les séquences de l'invention sont exprimées dans la souche d'*E.coli* TG1 sous forme de protéines de fusion avec la glutathion S-transférase (GST) selon la technique décrite par Smith et Johnson [Gene 67 (1988) 31]. Pour cela, les différents fragments d'ADN décrits en 3.1. ci-dessus ont été clonés, sous forme de fragments SmaI-EcoRI, dans le vecteur pGEX 2T (Pharmacia), en 3' et en phase d'un ADNc codant pour la GST. Les fragments SmaI-EcoRI sont obtenus par ajout d'un adaptateur au moyen d'une ligase. Les vecteurs ainsi obtenus sont ensuite utilisés pour transformer la souche *E.coli* TG1. Les cellules ainsi transformées sont précultivées une nuit à 37°C, diluées au 1/10e dans du milieu LB, ajoutées d'IPTG pour induire l'expression (2 heures, 25°C), puis cultivées 21 heures environ à 25°C. Les cellules sont ensuite lysées, et les protéines de fusions produites sont purifiées par affinité sur colonne Agarose-GSH. Pour cela, le lysat bactérien est incubé en présence du gel (préparé et équilibré avec le tampon de lyse) pendant 15 minutes à 4°C. Après 3 lavages avec un tampon Tris-HCl pH 7,4, les protéines sont éluées en présence d'un tampon Tris-HCl pH 7,7 contenant un excès de GSH. Le surnageant est récolté et centrifugé.

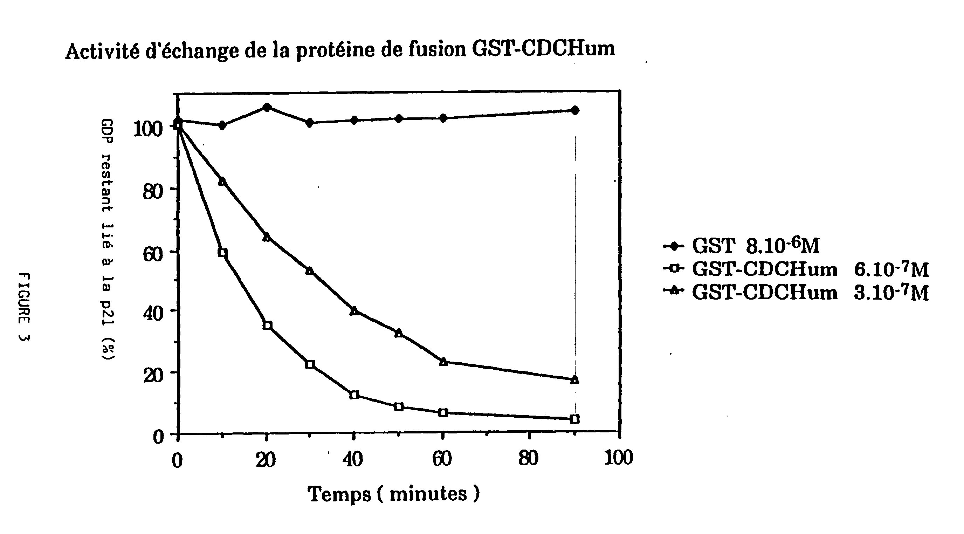

L'activité d'échange du GDP des peptides de l'invention sur des protéines Ha-Ras purifiées a ensuite été démontrée *in vitro* selon le protocole décrit par Rey et al. (Mol. Cell. Biol. précitée). Les résultats obtenus sont présentés sur la figure 3. Ils montrent notamment que le peptide de l'invention correspondant à la séquence CDC hum exprimée pae le fragment PstI-EcoRI décrit ci-avant stimule l'échange du GDP.

### 4. Mise en évidence de séquences homologues

Des séquences d'acides nucléiques homologues à celle présentée sur la figure 1 ont été mises en évidence par deux stratégies différentes :
- par PCR, dans les conditions décrites dans les techniques générales de clonage, sur des ADNc néosynthétisés à partir d'ARNm de placenta, en utilisant comme amorces des oligonucléotides dégénérés choisis pour recouvrir des séquences conservées entre la séquence SEQ ID n°1 et la séquence de la protéine SOS [Bonfini et al., Science 255 (1992) 603].
- Par criblage d'une banque d'ADNc de placenta à l'aide d'une sonde constituée par la totalité de la séquence SEQ ID n°1 marquée au ³²P. Ce criblage a été réalisé dans des conditions de faible stringence : hybridation à 50°C en milieu 5 x SSC, 5 x Denhart's; puis lavage à 50°C en milieu 2 x SSC.

Ces deux stratégies ont permis de révéler des séquences homologues à la séquence SEQ ID n°1. Ces séquences peuvent être aisément isolées et caractérisées. Elles constituent des séquences nucléiques au sens de la présente invention, lorsqu'elles codent (ou leurs fragments ou dérivés) pour des peptides capables de moduler les niveaux d'échanges du GDP sur des complexes p21-GDP.

En particulier, cette stratégie a permis l'identification, à partir d'ARNm de placenta et en utilisant les oligonucléotides oligo 2449 (SEQ ID n° 9) et oligo 2451 (SEQ ID n° 10), de 2 ADNc codant pour des facteurs désignés hSOS1 et hSOS2, dont les séquences partielles sont représentées sur les SEQ ID n° 5 et SEQ ID n° 7 respectivement. Les ARNm correspondant à ces facteurs sont présents dans tous les tissus dans lesquels ils ont été recherchés, contrairement au facteur décrit dans l'exemple 1 qui semble localisé uniquement dans le cerveau. La localisation chromosomique de ces gènes a été effectuée et a donné les résultats suivants :
- h-GRF : 15q2.4.
- h-SOS1 : 4q2.1.
- h-SOS2 : 14q2.2.

### 5. Recherche de facteurs d'échange de type h-GRF dans d'autres tissus

Un anticorps anti h-GRF a été préparé chez le lapin, par immunisation avec un antigène correspondant au fragment de 280 acides aminés localisé entre les résidus 211 et 489 du facteur h-GRF présenté sur la SEQ ID n° 4.

Cet anticorps a permis la détection, par ELISA, dans du cortex humain et des cellules précurseur du cerveau, de protéines de poids moléculaires apparents 30, 55, 75, 95 et 140 kDa. La diversité des poids moléculaires suggère la présence d'ADNc multiples. La préincubation de l'anticorps anti h-GRF avec le h-GRF supprime la détection des protéines identifiées, ce qui démontre la spécificité du signal.

### LISTE DE SEQUENCES

(1) INFORMATION GENERALE:
   (i) DEPOSANT:
      (A) NOM: RHONE-POULENC RORER S.A.
      (B) RUE: 20, avenue Raymond ARON
      (C) VILLE: ANTONY
      (E) PAYS: FRANCE
      (F) CODE POSTAL: 92165
   (ii) TITRE DE L' INVENTION: PEPTIDES INHIBANT L'ACTIVITE DES PROTEINES RAS, PREPARATION ET UTILISATION
   (iii) NOMBRE DE SEQUENCES: 12
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 2652 paires de bases
      (B) TYPE: acide nucléque
      (C) NOMBRE DE BRINS: double
      (D) CONFIGURATION: linéire
   (ii) TYPE DE MOLECULE: ADNc
   (iii) HYPOTHETIQUE: NON
   (iii) ANTI-SENS: NON
   (ix) CARACTERISTIQUE ADDITIONELLE:
      (A) NOM/CLE: CDS
      (B) EMPLACEMENT: 1..2445
   (ix) CARACTERISTIQUE ADDITIONELLE:
      (A) NOM/CLE: CDS
      (B) EMPLACEMENT: 445..2445 (SEQ ID NO 3)
   (ix) CARACTERISTIQUE ADDITIONELLE:
      (A) NOM/CLE: CDS
      (B) EMPLACEMENT: 976..2445 (SEQ ID NO 4)
   (xi) DESCRIPTION DE LA SEQUENCE: SEQ ID NO: 1 :
   GGC GAT GGC TGT AAG ATC CTC CTG GAC ACC AGC CAG ACC TTT GTG AGA        48
   Gly Asp Gly Cys Lys Ile Leu Leu Asp Thr Ser Gln Thr Phe Val Arg
    1               5                   10                  15
   CAA GGT TCC CTC ATT CAG GTG CCC ATG TCT GAA AAG GGC AAG ATC ACC        96
   Gln Gly Ser Leu Ile Gln Val Pro Met Ser Glu Lys Gly Lys Ile Thr
               20                  25                  30
   AGG GGG CGC CTG GGG TCT CTC TCC CTA AAG AAA GAG GGC GAG CGA CAG       144
   Arg Gly Arg Leu Gly Ser Leu Ser Leu Lys Lys Glu Gly Glu Arg Gln
           35                  40                  45
   TGC TTC CTG TTT TCT AAG CAT CTG ATT ATC TGT ACC AGA GGC TCT GGA      192
   Cys Phe Leu Phe Ser Lys His Leu Ile Ile Cys Thr Arg Gly Ser Gly
       50                  55                  60
   GGG AAG CTT CAC TTG ACC AAG AAT GGA GTC ATA TCC CTC ATT GAC TGC      240
   Gly Lys Leu His Leu Thr Lys Asn Gly Val Ile Ser Leu Ile Asp Cys
   65                  70                  75                  80
   ACT TTA TTG GAG GAG CCA GAA AGC ACG GAG GAG GAA GCC AAA GGA TCC      288
   Thr Leu Leu Glu Glu Pro Glu Ser Thr Glu Glu Glu Ala Lys Gly Ser
                   85                  90                  95
   GGC CAA GAC ATA GAT CAC TTG GAT TTT AAA ATC GGG GTG GAG CCA AAG      336
   Gly Gln Asp Ile Asp His Leu Asp Phe Lys Ile Gly Val Glu Pro Lys
               100                 105                 110
   GAT TCC CCG CCC TTT ACA GTC ATC CTA GTG GCC TCG TCC AGA CAG GAG      384
   Asp Ser Pro Pro Phe Thr Val Ile Leu Val Ala Ser Ser Arg Gln Glu
           115                 120                 125
   AAG GCA GCG TGG ACC AGT GAC ATC AGC CAG TGT GTT GGT AAC ATC CGA      432
   Lys Ala Ala Trp Thr Ser Asp Ile Ser Gln Cys Val Gly Asn Ile Arg
       130                 135                 140
   TGC AAT GGG CTC ATG ATG AAG CCA TTT GAA GAA AAT TCC AAG GTC ACT      480
   Cys Asn Gly Leu Met Met Lys Pro Phe Glu Glu Asn Ser Lys Val Thr
   145                 150                 155                 160
   GTG CCG CAG ATG ATC AAG TCC GAC GCC TCC TTA TAT TGT GAT GAT GTT      528
   Val Pro Gln Met Ile Lys Ser Asp Ala Ser Leu Tyr Cys Asp Asp Val
                   165                 170                 175
   GAC ATT CGC TTC AGC AAA ACC ATG AAC TCC TGC AAA GTG CTG CAG ATC      576
   Asp Ile Arg Phe Ser Lys Thr Met Asn Ser Cys Lys Val Leu Gln Ile
               180                 185                 190
   GCC TAC GCC AGT GTG GAG CGG CTG CTG GAG AGG CTG ACG GAC CTG CGC      624
   Ala Tyr Ala Ser Val Glu Arg Leu Leu Glu Arg Leu Thr Asp Leu Arg
           195                 200                 205
   TTC CTG AGC ATC GAC TTC CTC AAC ACC TTC CTG CAC TCC TAC CGC GTC      672
   Phe Leu Ser Ile Asp Phe Leu Asn Thr Phe Leu His Ser Tyr Arg Val
       210                 215                 220
   TTC ACC ACC GCC ATC GTG GTC CTG GAC AAG CTC ATT ACC ATC TAC AAG      720
   Phe Thr Thr Ala Ile Val Val Leu Asp Lys Leu Ile Thr Ile Tyr Lys
   225                 230                 235                 240
   AAG CCT ATC AGT GCC ATT CCT GCC AGG TCG CTG GAG CTC CTG TTT GCC      768
   Lys Pro Ile Ser Ala Ile Pro Ala Arg Ser Leu Glu Leu Leu Phe Ala
                   245                 250                 255
   AGT GGC CAG AAC AAT AAG CTC CTG TAC GGT GAA CCC CCC AAG TCC CCG      816
   Ser Gly Gln Asn Asn Lys Leu Leu Tyr Gly Glu Pro Pro Lys Ser Pro
               260                 265                 270
   CGC GCC ACC CGC AAG TTC TCC TCG CCG CCA CCT CTG TCC ATC ACC AAG      864
   Arg Ala Thr Arg Lys Phe Ser Ser Pro Pro Pro Leu Ser Ile Thr Lys
           275                 280                 285
   ACA TCG TCA CCG AGC CGC CGG CGG AAG CTC TCC CTG AAC ATC CCC ATC      912
   Thr Ser Ser Pro Ser Arg Arg Arg Lys Leu Ser Leu Asn Ile Pro Ile
       290                 295                 300
   ATC ACT GGC GGC AAG GCC CTG GAC CTG GCC GCC CTC AGC TGC AAC TCC      960
   Ile Thr Gly Gly Lys Ala Leu Asp Leu Ala Ala Leu Ser Cys Asn Ser
   305                 310                 315                 320
   AAT GGC TAC ACC AGC ATG TAC TCG GCC ATG TCA CCC TTC AGC AAG GCC     1008
   Asn Gly Tyr Thr Ser Met Tyr Ser Ala Met Ser Pro Phe Ser Lys Ala
                   325                 330                 335
   ACG CTG GAC ACC AGC AAG CTC TAT GTG TCC AGC AGC TTC ACC AAC AAG     1056
   Thr Leu Asp Thr Ser Lys Leu Tyr Val Ser Ser Ser Phe Thr Asn Lys
               340                 345                 350
   ATT CCA GAT GAG GGC GAT ACG ACC CCT GAG AAG CCC GAA GAC CCT TCA     1104
   Ile Pro Asp Glu Gly Asp Thr Thr Pro Glu Lys Pro Glu Asp Pro Ser
           355                 360                 365
   GCG CTC AGC AAG CAG AGC TCA GAA GTC TCC ATG AGA GAG GAG TCA GAT     1152
   Ala Leu Ser Lys Gln Ser Ser Glu Val Ser Met Arg Glu Glu Ser Asp
       370                 375                 380
   ATT GAT CAA AAC CAG AGT GAT GAT GGT GAT ACT GAA ACA TCA CCA ACT     1200
   Ile Asp Gln Asn Gln Ser Asp Asp Gly Asp Thr Glu Thr Ser Pro Thr
   385                 390                 395                 400
   AAA TCT CCA ACA ACA CCC AAA TCA GTC AAA AAC AAA AAT TCT TCA GAG     1248
   Lys Ser Pro Thr Thr Pro Lys Ser Val Lys Asn Lys Asn Ser Ser Glu
                   405                 410                 415
   TTC CCA CTC TTT TCC TAT AAC AAT GGA GTC GTC ATG ACC TCC TGT CGT     1296
   Phe Pro Leu Phe Ser Tyr Asn Asn Gly Val Val Met Thr Ser Cys Arg
               420                 425                 430
   GAA CTG GAC AAT AAC CGC AGT GCC TTG TCG GCC GCC TCT GCC TTT GCC     1344
   Glu Leu Asp Asn Asn Arg Ser Ala Leu Ser Ala Ala Ser Ala Phe Ala
           435                 440                 445
   ATA GCA ACC GCC GGG GCC AAC GAG GGC ACC CCA AAC AAG GAG AAG TAC     1392
   Ile Ala Thr Ala Gly Ala Asn Glu Gly Thr Pro Asn Lys Glu Lys Tyr
       450                 455                 460
   CGG AGG ATG TCC TTA GCC AGT GCA GGG TTT CCC CCA GAC CAG AGG AAT     1440
   Arg Arg Met Ser Leu Ala Ser Ala Gly Phe Pro Pro Asp Gln Arg Asn
   465                 470                 475                 480
   GGA GAC AAG GAG TTT GTG ATC CGC AGA GCA GCC ACC AAT CGT GTC TTG     1488
   Gly Asp Lys Glu Phe Val Ile Arg Arg Ala Ala Thr Asn Arg Val Leu
                   485                 490                 495
   AAC GTG CTC CGC CAC TGG GTG TCC AAG CAC TCT CAG GAC TTT GAG ACC     1536
   Asn Val Leu Arg His Trp Val Ser Lys His Ser Gln Asp Phe Glu Thr
               500                 505                 510
   AAC GAT GAG CTC AAA TGC AAG GTG ATC GGC TTC CTG GAA GAA GTC ATG     1584
   Asn Asp Glu Leu Lys Cys Lys Val Ile Gly Phe Leu Glu Glu Val Met
           515                 520                 525
   CAC GAC CCG GAG CTC CTG ACC CAG GAG CGG AAG GCT GCA GCC AAC ATC     1632
   His Asp Pro Glu Leu Leu Thr Gln Glu Arg Lys Ala Ala Ala Asn Ile
       530                 535                 540
   ATC AGG ACT CTG ACC CAG GAG GAC CCA GGT GAC AAC CAG ATC ACG CTG     1680
   Ile Arg Thr Leu Thr Gln Glu Asp Pro Gly Asp Asn Gln Ile Thr Leu
   545                 550                 555                 560
   GAG GAG ATC ACG CAG ATG GCT GAA GGC GTG AAG GCT GAG CCC TTT GAA     1728
   Glu Glu Ile Thr Gln Met Ala Glu Gly Val Lys Ala Glu Pro Phe Glu
                   565                 570                 575
   AAC CAC TCA GCC CTG GAG ATC GCG GAG CAG CTG ACC CTG CTA GAT CAC     1776
   Asn His Ser Ala Leu Glu Ile Ala Glu Gln Leu Thr Leu Leu Asp His
               580                 585                 590
   CTC GTC TTC AAG AAG ATT CCT TAT GAG GAG TTC TTC GGA CAA GGA TGG     1824
   Leu Val Phe Lys Lys Ile Pro Tyr Glu Glu Phe Phe Gly Gln Gly Trp
           595                 600                 605
   ATG AAA CTG GAA AAG AAT GAA AGG ACC CCT TAT ATC ATG AAA ACC ACT     1872
   Met Lys Leu Glu Lys Asn Glu Arg Thr Pro Tyr Ile Met Lys Thr Thr
       610                 615                 620
   AAG CAC TTC AAT GAC ATC AGT AAC TTG ATT GCT TCA GAA ATC ATC CGC     1920
   Lys His Phe Asn Asp Ile Ser Asn Leu Ile Ala Ser Glu Ile Ile Arg
   625                 630                 635                 640
   AAT GAG GAC ATC AAC GCC AGG GTG AGC GCC ATC GAG AAG TGG GTG GCC     1968
   Asn Glu Asp Ile Asn Ala Arg Val Ser Ala Ile Glu Lys Trp Val Ala
                   645                 650                 655
   GTA GCT GAC ATA TGC CGC TGC CTC CAC AAC TAC AAT GCC GTA CTG GAG     2016
   Val Ala Asp Ile Cys Arg Cys Leu His Asn Tyr Asn Ala Val Leu Glu
               660                 665                 670
   ATC ACC TCG TCC ATG AAC CGC AGT GCA ATC TTC CGG CTC AAA AAG ACG     2064
   Ile Thr Ser Ser Met Asn Arg Ser Ala Ile Phe Arg Leu Lys Lys Thr
           675                 680                 685
   TGG CTC AAA GTC TCT AAG CAG ACT AAA GCT TTG ATT GAT AAG CTC CAA     2112
   Trp Leu Lys Val Ser Lys Gln Thr Lys Ala Leu Ile Asp Lys Leu Gln
       690                 695                 700
   AAG CTT GTG TCA TCT GAG GGC AGA TTT AAG AAT CTC AGA GAA GCT TTG     2160
   Lys Leu Val Ser Ser Glu Gly Arg Phe Lys Asn Leu Arg Glu Ala Leu
   705                 710                 715                 720
   AAA AAT TGT GAC CCA CCC TGT GTC CCT TAC CTG GGG ATG TAC CTC ACC     2208
   Lys Asn Cys Asp Pro Pro Cys Val Pro Tyr Leu Gly Met Tyr Leu Thr
                   725                 730                 735
   GAC CTG GCC TTC ATC GAG GAG GGG ACG CCC AAT TAC ACG GAA GAC GGC     2256
   Asp Leu Ala Phe Ile Glu Glu Gly Thr Pro Asn Tyr Thr Glu Asp Gly
               740                 745                 750
   CTG GTC AAC TTC TCC AAG ATG AGG ATG ATA TCC CAT ATT ATC CGA GAG     2304
   Leu Val Asn Phe Ser Lys Met Arg Met Ile Ser His Ile Ile Arg Glu
           755                 760                 765
   ATT CGC CAG TTT CAA CAA ACT GCC TAC AAA ATA GAG CAC CAA GCA AAG     2352
   Ile Arg Gln Phe Gln Gln Thr Ala Tyr Lys Ile Glu His Gln Ala Lys
       770                 775                 780
   GTA ACG CAA TAT TTA CTG GAC CAA TCT TTT GTA ATG GAT GAA GAA AGC     2400
   Val Thr Gln Tyr Leu Leu Asp Gln Ser Phe Val Met Asp Glu Glu Ser
   785                 790                 795                 800
   CTC TAC GAG TCT TCT CTC CGA ATA GAA CCA AAA CTC CCC ACC TGAAGCTGTG   2452
   Leu Tyr Glu Ser Ser Leu Arg Ile Glu Pro Lys Leu Pro Thr
                   805                 810                 815
   CCCAGCCCAG ACCCAGCTGC TCCCGGGGAC ATGTGCTAGA TGATACTGTA CATATTCGTT   2512
   TGGTTTCACT GGATTTTCTT CTTCAGTATG TGCTTCTCCA AGAATACAAA TCGTCCTTGT   2572
   TCTTAGATTC CTGTAGAACC GGAATATGAA TTTCTGCACC GTTTCAGACT TCGCCCACCC   2632
   ATCCCTCCCC TCGCCCGAAT                                                2652
(2) INFORMATION POUR LA SEQ ID NO: 2:
   (i) CARACTERISTIQUES DE LA SEQUENCE:
      (A) LONGUEUR: 814 acides aminé
      (B) TYPE: acide aminé
      (D) CONFIGURATION: linéire
   (ii) TYPE DE MOLECULE: proténe
   (xi) DESCRIPTION DE LA SEQUENCE: SEQ ID NO: 2:
   Gly Asp Gly Cys Lys Ile Leu Leu Asp Thr Ser Gln Thr Phe Val Arg
    1               5                  10                  15
   Gln Gly Ser Leu Ile Gln Val Pro Met Ser Glu Lys Gly Lys Ile Thr
               20                  25                  30
   Arg Gly Arg Leu Gly Ser Leu Ser Leu Lys Lys Glu Gly Glu Arg Gln
           35                  40                  45
   Cys Phe Leu Phe Ser Lys His Leu Ile Ile Cys Thr Arg Gly Ser Gly
       50                  55                  60
   Gly Lys Leu His Leu Thr Lys Asn Gly Val Ile Ser Leu Ile Asp Cys
   65                  70                  75                  80
   Thr Leu Leu Glu Glu Pro Glu Ser Thr Glu Glu Glu Ala Lys Gly Ser
                   85                  90                  95
   Gly Gln Asp Ile Asp His Leu Asp Phe Lys Ile Gly Val Glu Pro Lys
               100                 105                 110
   Asp Ser Pro Pro Phe Thr Val Ile Leu Val Ala Ser Ser Arg Gln Glu
           115                 120                 125
   Lys Ala Ala Trp Thr Ser Asp Ile Ser Gln Cys Val Gly Asn Ile Arg
       130                 135                 140
   Cys Asn Gly Leu Met Met Lys Pro Phe Glu Glu Asn Ser Lys Val Thr
   145                 150                 155                 160
   Val Pro Gln Met Ile Lys Ser Asp Ala Ser Leu Tyr Cys Asp Asp Val
                   165                 170                 175
   Asp Ile Arg Phe Ser Lys Thr Met Asn Ser Cys Lys Val Leu Gln Ile
               180                 185                 190
   Ala Tyr Ala Ser Val Glu Arg Leu Leu Glu Arg Leu Thr Asp Leu Arg
           195                     200                     205
   Phe Leu Ser Ile Asp Phe Leu Asn Thr Phe Leu His Ser Tyr Arg Val
       210                 215                 220
   Phe Thr Thr Ala Ile Val Val Leu Asp Lys Leu Ile Thr Ile Tyr Lys
   225                 230                 235                 240
   Lys Pro Ile Ser Ala Ile Pro Ala Arg Ser Leu Glu Leu Leu Phe Ala
                   245                 250                 255
   Ser Gly Gln Asn Asn Lys Leu Leu Tyr Gly Glu Pro Pro Lys Ser Pro
               260                 265                 270
   Arg Ala Thr Arg Lys Phe Ser Ser Pro Pro Pro Leu Ser Ile Thr Lys
           275                 280                 285
   Thr Ser Ser Pro Ser Arg Arg Arg Lys Leu Ser Leu Asn Ile Pro Ile
       290                 295                 300
   Ile Thr Gly Gly Lys Ala Leu Asp Leu Ala Ala Leu Ser Cys Asn Ser
   305                 310                 315                 320
   Asn Gly Tyr Thr Ser Met Tyr Ser Ala Met Ser Pro Phe Ser Lys Ala
                   325                 330                 335
   Thr Leu Asp Thr Ser Lys Leu Tyr Val Ser Ser Ser Phe Thr Asn Lys
               340                 345                 350
   Ile Pro Asp Glu Gly Asp Thr Thr Pro Glu Lys Pro Glu Asp Pro Ser
           355                 360                 365
   Ala Leu Ser Lys Gln Ser Ser Glu Val Ser Met Arg Glu Glu Ser Asp
       370                 375                 380
   Ile Asp Gln Asn Gln Ser Asp Asp Gly Asp Thr Glu Thr Ser Pro Thr
   385                 390                 395                 400
   Lys Ser Pro Thr Thr Pro Lys Ser Val Lys Asn Lys Asn Ser Ser Glu
                   405                 410                 415
   Phe Pro Leu Phe Ser Tyr Asn Asn Gly Val Val Met Thr Ser Cys Arg
               420                 425                 430
   Glu Leu Asp Asn Asn Arg Ser Ala Leu Ser Ala Ala Ser Ala Phe Ala
           435                 440                 445
   Ile Ala Thr Ala Gly Ala Asn Glu Gly Thr Pro Asn Lys Glu Lys Tyr
       450                 455                 460
   Arg Arg Met Ser Leu Ala Ser Ala Gly Phe Pro Pro Asp Gln Arg Asn
   465                 470                 475                 480
   Gly Asp Lys Glu Phe Val Ile Arg Arg Ala Ala Thr Asn Arg Val Leu
                   485                 490                 495
   Asn Val Leu Arg His Trp Val Ser Lys His Ser Gln Asp Phe Glu Thr
               500                 505                 510
   Asn Asp Glu Leu Lys Cys Lys Val Ile Gly Phe Leu Glu Glu Val Met
           515                 520                 525
   His Asp Pro Glu Leu Leu Thr Gln Glu Arg Lys Ala Ala Ala Asn Ile
                       530                     535                     540
   Ile Arg Thr Leu Thr Gln Glu Asp Pro Gly Asp Asn Gln Ile Thr Leu
   545                 550                 555                 560
   Glu Glu Ile Thr Gln Met Ala Glu Gly Val Lys Ala Glu Pro Phe Glu
                   565                 570                 575
   Asn His Ser Ala Leu Glu Ile Ala Glu Gln Leu Thr Leu Leu Asp His
               580                 585                 590
   Leu Val Phe Lys Lys Ile Pro Tyr Glu Glu Phe Phe Gly Gln Gly Trp
           595                 600                 605
   Met Lys Leu Glu Lys Asn Glu Arg Thr Pro Tyr Ile Met Lys Thr Thr
       610                 615                 620
   Lys His Phe Asn Asp Ile Ser Asn Leu Ile Ala Ser Glu Ile Ile Arg
   625                 630                 635                 640
   Asn Glu Asp Ile Asn Ala Arg Val Ser Ala Ile Glu Lys Trp Val Ala
                   645                 650                 655
   Val Ala Asp Ile Cys Arg Cys Leu His Asn Tyr Asn Ala Val Leu Glu
               660                 665                 670
   Ile Thr Ser Ser Met Asn Arg Ser Ala Ile Phe Arg Leu Lys Lys Thr
           675                 680                 685
   Trp Leu Lys Val Ser Lys Gln Thr Lys Ala Leu Ile Asp Lys Leu Gln
       690                 695                 700
   Lys Leu Val Ser Ser Glu Gly Arg Phe Lys Asn Leu Arg Glu Ala Leu
   705                 710                 715                 720
   Lys Asn Cys Asp Pro Pro Cys Val Pro Tyr Leu Gly Met Tyr Leu Thr
                   725                 730                 735
   Asp Leu Ala Phe Ile Glu Glu Gly Thr Pro Asn Tyr Thr Glu Asp Gly
               740                 745                 750
   Leu Val Asn Phe Ser Lys Met Arg Met Ile Ser His Ile Ile Arg Glu
           755                 760                 765
   Ile Arg Gln Phe Gln Gln Thr Ala Tyr Lys Ile Glu His Gln Ala Lys
       770                 775                 780
   Val Thr Gln Tyr Leu Leu Asp Gln Ser Phe Val Met Asp Glu Glu Ser
   785                 790                 795                 800
   Leu Tyr Glu Ser Ser Leu Arg Ile Glu Pro Lys Leu Pro Thr
                   805                 810
(2) INFORMATION POUR LA SEQ ID NO: 3:
   (i) CARACTERISTIQUES DE LA SEQUENCE:
      (A) LONGUEUR: 666 acides aminé
      (B) TYPE: acide aminé
      (D) CONFIGURATION: linéire
   (ii) TYPE DE MOLECULE: proténe
   (xi) DESCRIPTION DE LA SEQUENCE: SEQ ID NO: 3:
   Met Met Lys Pro Phe Glu Glu Asn Ser Lys Val Thr Val Pro Gln Met
   1 5 10 15
   Ile Lys Ser Asp Ala Ser Leu Tyr Cys Asp Asp Val Asp Ile Arg Phe
   20 25 30
   Ser Lys Thr Met Asn Ser Cys Lys Val Leu Gln Ile Ala Tyr Ala Ser
   35 40 45
   Val Glu Arg Leu Leu Glu Arg Leu Thr Asp Leu Arg Phe Leu Ser Ile
   50 55 60
   Asp Phe Leu Asn Thr Phe Leu His Ser Tyr Arg Val Phe Thr Thr Ala
   65 70 75 80
   Ile Val Val Leu Asp Lys Leu Ile Thr Ile Tyr Lys Lys Pro Ile Ser
   85 90 95
   Ala Ile Pro Ala Arg Ser Leu Glu Leu Leu Phe Ala Ser Gly Gln Asn
   100 105 110
   Asn Lys Leu Leu Tyr Gly Glu Pro Pro Lys Ser Pro Arg Ala Thr Arg
   115 120 125
   Lys Phe Ser Ser Pro Pro Pro Leu Ser Ile Thr Lys Thr Ser Ser Pro
   130 135 140
   Ser Arg Arg Arg Lys Leu Ser Leu Asn Ile Pro Ile Ile Thr Gly Gly
   145 150 155 160
   Lys Ala Leu Asp Leu Ala Ala Leu Ser Cys Asn Ser Asn Gly Tyr Thr
   165 170 175
   Ser Met Tyr Ser Ala Met Ser Pro Phe Ser Lys Ala Thr Leu Asp Thr
   180 185 190
   Ser Lys Leu Tyr Val Ser Ser Ser Phe Thr Asn Lys Ile Pro Asp Glu
   195 200 205
   Gly Asp Thr Thr Pro Glu Lys Pro Glu Asp Pro Ser Ala Leu Ser Lys
   210 215 220
   Gln Ser Ser Glu Val Ser Met Arg Glu Glu Ser Asp Ile Asp Gln Asn
   225 230 235 240
   Gln Ser Asp Asp Gly Asp Thr Glu Thr Ser Pro Thr Lys Ser Pro Thr
   245 250 255
   Thr Pro Lys Ser Val Lys Asn Lys Asn Ser Ser Glu Phe Pro Leu Phe
   260 265 270
   Ser Tyr Asn Asn Gly Val Val Met Thr Ser Cys Arg Glu Leu Asp Asn
   275 280 285
   Asn Arg Ser Ala Leu Ser Ala Ala Ser Ala Phe Ala Ile Ala Thr Ala
   290 295 300
   Gly Ala Asn Glu Gly Thr Pro Asn Lys Glu Lys Tyr Arg Arg Met Ser
   305 310 315 320
   Leu Ala Ser Ala Gly Phe Pro Pro Asp Gln Arg Asn Gly Asp Lys Glu
                   325                 330                 335
   Phe Val Ile Arg Arg Ala Ala Thr Asn Arg Val Leu Asn Val Leu Arg
               340                 345                 350
   His Trp Val Ser Lys His Ser Gln Asp Phe Glu Thr Asn Asp Glu Leu
           355                 360                 365
   Lys Cys Lys Val Ile Gly Phe Leu Glu Glu Val Met His Asp Pro Glu
       370                 375                 380
   Leu Leu Thr Gln Glu Arg Lys Ala Ala Ala Asn Ile Ile Arg Thr Leu
   385                 390                 395                 400
   Thr Gln Glu Asp Pro Gly Asp Asn Gln Ile Thr Leu Glu Glu Ile Thr
                   405                 410                 415
   Gln Met Ala Glu Gly Val Lys Ala Glu Pro Phe Glu Asn His Ser Ala
               420                 425                 430
   Leu Glu Ile Ala Glu Gln Leu Thr Leu Leu Asp His Leu Val Phe Lys
           435                 440                 445
   Lys Ile Pro Tyr Glu Glu Phe Phe Gly Gln Gly Trp Met Lys Leu Glu
       450                 455                 460
   Lys Asn Glu Arg Thr Pro Tyr Ile Met Lys Thr Thr Lys His Phe Asn
   465                 470                 475                 480
   Asp Ile Ser Asn Leu Ile Ala Ser Glu Ile Ile Arg Asn Glu Asp Ile
                   485                 490                 495
   Asn Ala Arg Val Ser Ala Ile Glu Lys Trp Val Ala Val Ala Asp Ile
               500                 505                 510
   Cys Arg Cys Leu His Asn Tyr Asn Ala Val Leu Glu Ile Thr Ser Ser
           515                 520                 525
   Met Asn Arg Ser Ala Ile Phe Arg Leu Lys Lys Thr Trp Leu Lys Val
       530                 535                 540
   Ser Lys Gln Thr Lys Ala Leu Ile Asp Lys Leu Gln Lys Leu Val Ser
   545                 550                 555                 560
   Ser Glu Gly Arg Phe Lys Asn Leu Arg Glu Ala Leu Lys Asn Cys Asp
                   565                 570                 575
   Pro Pro Cys Val Pro Tyr Leu Gly Met Tyr Leu Thr Asp Leu Ala Phe
               580                 585                 590
   Ile Glu Glu Gly Thr Pro Asn Tyr Thr Glu Asp Gly Leu Val Asn Phe
           595                 600                 605
   Ser Lys Met Arg Met Ile Ser His Ile Ile Arg Glu Ile Arg Gln Phe
       610                 615                 620
   Gln Gln Thr Ala Tyr Lys Ile Glu His Gln Ala Lys Val Thr Gln Tyr
   625                 630                 635                 640
   Leu Leu Asp Gln Ser Phe Val Met Asp Glu Glu Ser Leu Tyr Glu Ser
                   645                 650                 655
   Ser Leu Arg Ile Glu Pro Lys Leu Pro Thr
   660 665
(2) INFORMATION POUR LA SEQ ID NO: 4:
   (i) CARACTERISTIQUES DE LA SEQUENCE:
      (A) LONGUEUR: 489 acides aminé
      (B) TYPE: acide aminé
      (D) CONFIGURATION: linéire
   (ii) TYPE DE MOLECULE: proténe
   (xi) DESCRIPTION DE LA SEQUENCE: SEQ ID NO: 4:
   Met Tyr Ser Ala Met Ser Pro Phe Ser Lys Ala Thr Leu Asp Thr Ser
   1 5 10 15
   Lys Leu Tyr Val Ser Ser Ser Phe Thr Asn Lys Ile Pro Asp Glu Gly
   20 25 30
   Asp Thr Thr Pro Glu Lys Pro Glu Asp Pro Ser Ala Leu Ser Lys Gln
   35 40 45
   Ser Ser Glu Val Ser Met Arg Glu Glu Ser Asp Ile Asp Gln Asn Gln
   50 55 60
   Ser Asp Asp Gly Asp Thr Glu Thr Ser Pro Thr Lys Ser Pro Thr Thr
   65 70 75 80
   Pro Lys Ser Val Lys Asn Lys Asn Ser Ser Glu Phe Pro Leu Phe Ser
   85 90 95
   Tyr Asn Asn Gly Val Val Met Thr Ser Cys Arg Glu Leu Asp Asn Asn
   100 105 110
   Arg Ser Ala Leu Ser Ala Ala Ser Ala Phe Ala Ile Ala Thr Ala Gly
   115 120 125
   Ala Asn Glu Gly Thr Pro Asn Lys Glu Lys Tyr Arg Arg Met Ser Leu
   130 135 140
   Ala Ser Ala Gly Phe Pro Pro Asp Gln Arg Asn Gly Asp Lys Glu Phe
   145 150 155 160
   Val Ile Arg Arg Ala Ala Thr Asn Arg Val Leu Asn Val Leu Arg His
   165 170 175
   Trp Val Ser Lys His Ser Gln Asp Phe Glu Thr Asn Asp Glu Leu Lys
   180 185 190
   Cys Lys Val Ile Gly Phe Leu Glu Glu Val Met His Asp Pro Glu Leu
   195 200 205
   Leu Thr Gln Glu Arg Lys Ala Ala Ala Asn Ile Ile Arg Thr Leu Thr
   210 215 220
   Gln Glu Asp Pro Gly Asp Asn Gln Ile Thr Leu Glu Glu Ile Thr Gln
   225 230 235 240
   Met Ala Glu Gly Val Lys Ala Glu Pro Phe Glu Asn His Ser Ala Leu
   245 250 255
   Glu Ile Ala Glu Gln Leu Thr Leu Leu Asp His Leu Val Phe Lys Lys
   260 265 270
   Ile Pro Tyr Glu Glu Phe Phe Gly Gln Gly Trp Met Lys Leu Glu Lys
   275 280 285
   Asn Glu Arg Thr Pro Tyr Ile Met Lys Thr Thr Lys His Phe Asn Asp
   290 295 300
   Ile Ser Asn Leu Ile Ala Ser Glu Ile Ile Arg Asn Glu Asp Ile Asn
   305 310 315 320
   Ala Arg Val Ser Ala Ile Glu Lys Trp Val Ala Val Ala Asp Ile Cys
   325 330 335
   Arg Cys Leu His Asn Tyr Asn Ala Val Leu Glu Ile Thr Ser Ser Met
   340 345 350
   Asn Arg Ser Ala Ile Phe Arg Leu Lys Lys Thr Trp Leu Lys Val Ser
   355 360 365
   Lys Gln Thr Lys Ala Leu Ile Asp Lys Leu Gln Lys Leu Val Ser Ser
   370 375 380
   Glu Gly Arg Phe Lys Asn Leu Arg Glu Ala Leu Lys Asn Cys Asp Pro
   385 390 395 400
   Pro Cys Val Pro Tyr Leu Gly Met Tyr Leu Thr Asp Leu Ala Phe Ile
   405 410 415
   Glu Glu Gly Thr Pro Asn Tyr Thr Glu Asp Gly Leu Val Asn Phe Ser
   420 425 430
   Lys Met Arg Met Ile Ser His Ile Ile Arg Glu Ile Arg Gln Phe Gln
   435 440 445
   Gln Thr Ala Tyr Lys Ile Glu His Gln Ala Lys Val Thr Gln Tyr Leu
   450 455 460
   Leu Asp Gln Ser Phe Val Met Asp Glu Glu Ser Leu Tyr Glu Ser Ser
   465 470 475 480
   Leu Arg Ile Glu Pro Lys Leu Pro Thr
   485
(2) INFORMATION POUR LA SEQ ID NO: 5:
   (i) CARACTERISTIQUES DE LA SEQUENCE:
      (A) LONGUEUR: 1092 paires de bases
      (B) TYPE: acide nucléque
      (C) NOMBRE DE BRINS: double
      (D) CONFIGURATION: linéire
   (ii) TYPE DE MOLECULE: ADNc
   (iii) HYPOTHETIQUE: NON
   (iii) ANTI-SENS: NON
   (ix) CARACTERISTIQUE ADDITIONELLE:
      (A) NOM/CLE: CDS
      (B) EMPLACEMENT: 1..1092
   (xi) DESCRIPTION DE LA SEQUENCE: SEQ ID NO: 5:
   ATT ACT AAA ATA ATC CAA AGG AAA AAA ATT GCA AGA GAC AAT GGA CCA      48
   Ile Thr Lys Ile Ile Gln Arg Lys Lys Ile Ala Arg Asp Asn Gly Pro
    1               5                  10                  15
   GGT CAT AAT ATT ACA TTT CAG AGT TCA CCT CCC ACA GTT GAG TGG CAT      96
   Gly His Asn Ile Thr Phe Gln Ser Ser Pro Pro Thr Val Glu Trp His
               20                  25                  30
   ATA AGC AGA CCT GGG CAC ATA GAG ACT TTT GAC CTG CTC ACC TTA CAC     144
   Ile Ser Arg Pro Gly His Ile Glu Thr Phe Asp Leu Leu Thr Leu His
           35                  40                  45
   CCA ATA GAA ATT GCT CGA CAA CTC ACT TTA CTT GAT TCA GAT CTA TAC     192
   Pro Ile Glu Ile Ala Arg Gln Leu Thr Leu Leu Asp Ser Asp Leu Tyr
       50                  55                  60
   CGA GCT GTA CAG CCA TCA GAT TTA GTT GGA AGT GTG TGG ACA AAA GAA     240
   Arg Ala Val Gln Pro Ser Asp Leu Val Gly Ser Val Trp Thr Lys Glu
   65                  70                  75                  80
   GAC AAA GAA ATT AAC TCT CCT AAT CTT CTG AAA ATG ATT CGA CAT ACC     288
   Asp Lys Glu Ile Asn Ser Pro Asn Leu Leu Lys Met Ile Arg His Thr
                   85                  90                  95
   ACC AAC CTC ACT CTG TGG TTT GAG AAA TGT ATT GTA GAA ACT GAA AAT     336
   Thr Asn Leu Thr Leu Trp Phe Glu Lys Cys Ile Val Glu Thr Glu Asn
               100                 105                 110
   TTA GAA GAA AGA GTA GCT GTG GTG AGT CGA ATT ATT GAG ATT CTA CAA     384
   Leu Glu Glu Arg Val Ala Val Val Ser Arg Ile Ile Glu Ile Leu Gln
           115                 120                 125
   GTC TTT CAA GAG TTG AAC AAC TTT AAT GGG GTC CTT GAG GTT GTC AGT     432
   Val Phe Gln Glu Leu Asn Asn Phe Asn Gly Val Leu Glu Val Val Ser
       130                 135                 140
   GCT ATG AAT TCC TCA CCT GTT TAC AGA CTA GAC CAC ACA TTT GAG CAA     480
   Ala Met Asn Ser Ser Pro Val Tyr Arg Leu Asp His Thr Phe Glu Gln
   145                 150                 155                 160
   ATA CCA AGT CGC CAG AAG AAA ATT TTA GAA GAA GCT CAT GAA TTG AGT     528
   Ile Pro Ser Arg Gln Lys Lys Ile Leu Glu Glu Ala His Glu Leu Ser
                   165                 170                 175
   GAA GAT CAC TAT AAG AAA TAT TTG GCA AAA CTC AGG TCT ATT AAT CCA     576
   Glu Asp His Tyr Lys Lys Tyr Leu Ala Lys Leu Arg Ser Ile Asn Pro
               180                 185                 190
   CCA TGT GTG CCT TTC TTT GGA ATT TAT CTA CAT AAT ATC TTG AAA ACA     624
   Pro Cys Val Pro Phe Phe Gly Ile Tyr Leu His Asn Ile Leu Lys Thr
           195                 200                 205
   GAA GAA GGC AAC CCT GAG GTC CTA AAA AGA CAT GGA AAA GAG CTT ATA     672
   Glu Glu Gly Asn Pro Glu Val Leu Lys Arg His Gly Lys Glu Leu Ile
       210                 215                 220
   AAC TTT AGC AAA AGG AGG AAA GTA GCA GAA ATA ACA GGA GAG ATC CAG     720
   Asn Phe Ser Lys Arg Arg Lys Val Ala Glu Ile Thr Gly Glu Ile Gln
   225                 230                 235                 240
   CAG TAC CAA AAT CAG CCT TAC TGT TTA CGA GTA GAA TCA GAT ATC AAA      768
   Gln Tyr Gln Asn Gln Pro Tyr Cys Leu Arg Val Glu Ser Asp Ile Lys
                   245                 250                 255
   AGG TTC TTT GAA AAC TTG AAT CCG ATG GGA AAT AGC ATG GAG AGG GAA      816
   Arg Phe Phe Glu Asn Leu Asn Pro Met Gly Asn Ser Met Glu Arg Glu
               260                 265                 270
   TTT ACA GAT TAT CTT TTC AAC AAA TCC CTA GAA ATA GAA CCA CGA AAC      864
   Phe Thr Asp Tyr Leu Phe Asn Lys Ser Leu Glu Ile Glu Pro Arg Asn
           275                 280                 285
   CCT AAG CCT CTC CCA AGA TTT CCA AAA AAA TAT ACG TAT CCC CTA AAA      912
   Pro Lys Pro Leu Pro Arg Phe Pro Lys Lys Tyr Thr Tyr Pro Leu Lys
       290                 295                 300
   TCT CCT GGT GTC CGG CCA TCA AAC CCA AGA CCG GGT ACC ATG AGG ATC      960
   Ser Pro Gly Val Arg Pro Ser Asn Pro Arg Pro Gly Thr Met Arg Ile
   305                 310                 315                 320
   CCC ACC CCT CTA CAG CAG GAA CCA CGA AAA ATA AGT TAT AGT AGA ATA     1008
   Pro Thr Pro Leu Gln Gln Glu Pro Arg Lys Ile Ser Tyr Ser Arg Ile
                   325                 330                 335
   CCA GAG TCA GAG ACA GAG AGT ACT GCT AGT GCA CCT AAT TCA CCA AGG     1056
   Pro Glu Ser Glu Thr Glu Ser Thr Ala Ser Ala Pro Asn Ser Pro Arg
               340                 345                 350
   ACA CCT CTA ACA CCT CCA CCT GCA TCA GGA ACA TCA                     1092
   Thr Pro Leu Thr Pro Pro Pro Ala Ser Gly Thr Ser
           355                 360
(2) INFORMATION POUR LA SEQ ID NO: 6:
   (i) CARACTERISTIQUES DE LA SEQUENCE:
      (A) LONGUEUR: 364 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 6:
   Ile Thr Lys Ile Ile Gln Arg Lys Lys Ile Ala Arg Asp Asn Gly Pro
   1               5                   10                  15
   Gly His Asn Ile Thr Phe Gln Ser Ser Pro Pro Thr Val Glu Trp His
               20                  25                  30
   Ile Ser Arg Pro Gly His Ile Glu Thr Phe Asp Leu Leu Thr Leu His
           35                  40                  45
   Pro Ile Glu Ile Ala Arg Gln Leu Thr Leu Leu Asp Ser Asp Leu Tyr
       50                  55                  60
   Arg Ala Val Gln Pro Ser Asp Leu Val Gly Ser Val Trp Thr Lys Glu
   65                  70                  75                  80
   Asp Lys Glu Ile Asn Ser Pro Asn Leu Leu Lys Met Ile Arg His Thr
                   85                  90                  95
   Thr Asn Leu Thr Leu Trp Phe Glu Lys Cys Ile Val Glu Thr Glu Asn
               100                 105                 110
   Leu Glu Glu Arg Val Ala Val Val Ser Arg Ile Ile Glu Ile Leu Gln
           115                 120                 125
   Val Phe Gln Glu Leu Asn Asn Phe Asn Gly Val Leu Glu Val Val Ser
       130                 135                 140
   Ala Met Asn Ser Ser Pro Val Tyr Arg Leu Asp His Thr Phe Glu Gln
   145                 150                 155                 160
   Ile Pro Ser Arg Gln Lys Lys Ile Leu Glu Glu Ala His Glu Leu Ser
                   165                 170                 175
   Glu Asp His Tyr Lys Lys Tyr Leu Ala Lys Leu Arg Ser Ile Asn Pro
               180                 185                 190
   Pro Cys Val Pro Phe Phe Gly Ile Tyr Leu His Asn Ile Leu Lys Thr
           195                 200                 205
   Glu Glu Gly Asn Pro Glu Val Leu Lys Arg His Gly Lys Glu Leu Ile
       210                 215                 220
   Asn Phe Ser Lys Arg Arg Lys Val Ala Glu Ile Thr Gly Glu Ile Gln
   225                 230                 235                 240
   Gln Tyr Gln Asn Gln Pro Tyr Cys Leu Arg Val Glu Ser Asp Ile Lys
                   245                 250                 255
   Arg Phe Phe Glu Asn Leu Asn Pro Met Gly Asn Ser Met Glu Arg Glu
               260                 265                 270
   Phe Thr Asp Tyr Leu Phe Asn Lys Ser Leu Glu Ile Glu Pro Arg Asn
           275                 280                 285
   Pro Lys Pro Leu Pro Arg Phe Pro Lys Lys Tyr Thr Tyr Pro Leu Lys
       290                 295                 300
   Ser Pro Gly Val Arg Pro Ser Asn Pro Arg Pro Gly Thr Met Arg Ile
   305                 310                 315                 320
   Pro Thr Pro Leu Gln Gln Glu Pro Arg Lys Ile Ser Tyr Ser Arg Ile
                   325                 330                 335
   Pro Glu Ser Glu Thr Glu Ser Thr Ala Ser Ala Pro Asn Ser Pro Arg
               340                 345                 350
   Thr Pro Leu Thr Pro Pro Pro Ala Ser Gly Thr Ser
           355                 360
(2) INFORMATION POUR LA SEQ ID NO: 7:
   (i) CARACTERISTIQUES DE LA SEQUENCE:
      (A) LONGUEUR: 1956 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (ix) CARACTERISTIQUE ADDITIONELLE:
      (A) NOM/CLE: CDS
      (B) EMPLACEMENT: 1..1956
   (xi) DESCRIPTION DE LA SEQUENCE: SEQ ID NO: 7:
   AGG TTT GAA ATC CCA GAG CCA GAA CCT ACA GAA GCA GAT AAA CTA GCA      48
   Arg Phe Glu Ile Pro Glu Pro Glu Pro Thr Glu Ala Asp Lys Leu Ala
    1               5                  10                  15
   CTT GAG AAA GGA GAA CAA CCA ATC TCT GCA GAT CTA AAG AGG TTC AGA      96
   Leu Glu Lys Gly Glu Gln Pro Ile Ser Ala Asp Leu Lys Arg Phe Arg
               20                  25                  30
   AAG GAA TAT ATC CAA CCA GTA CAG CTA CGG GTG TTG AAC GTG CAG CGG     144
   Lys Glu Tyr Ile Gln Pro Val Gln Leu Arg Val Leu Asn Val Gln Arg
           35                  40                  45
   CAC TGG GTT GAA CAT CAC CCC CAT GAC TTT GAA AGA GAC TTG GAA CTG     192
   His Trp Val Glu His His Pro His Asp Phe Glu Arg Asp Leu Glu Leu
       50                  55                  60
   CTC GAA AGA CTA GAA TCC TTC ACC TCA AGC GCT CAC AGA GCG AAA GCA     240
   Leu Glu Arg Leu Glu Ser Phe Thr Ser Ser Ala His Arg Ala Lys Ala
   65                  70                  75                  80
   ATG AAG AAG TGG GTA GAG AGC ATC GCT AAG ACC ATC AGG AGG AAG AAG     288
   Met Lys Lys Trp Val Glu Ser Ile Ala Lys Thr Ile Arg Arg Lys Lys
                   85                  90                  95
   CAA GCT CAG GCA AAT GGA GTA AGC CAT AAT ATT ACC TTT GAA AGT CCA     336
   Gln Ala Gln Ala Asn Gly Val Ser His Asn Ile Thr Phe Glu Ser Pro
               100                 105                 110
   CCT CCA CCA ATT GAA TGG CAT ATC AGC AAA CCA GGA CAG TTT GAA ACA     384
   Pro Pro Pro Ile Glu Trp His Ile Ser Lys Pro Gly Gln Phe Glu Thr
           115                 120                 125
   TTT GAT CTC ATG ACA CTT CAT CCA ATA GAA ATT GCA CGT CAG CTG ACA     432
   Phe Asp Leu Met Thr Leu His Pro Ile Glu Ile Ala Arg Gln Leu Thr
       130                 135                 140
   CTT TTG GAG TCT GAT CTT TAC AGG AAA GTT CAA CCG TCT GAA CTT GTA     480
   Leu Leu Glu Ser Asp Leu Tyr Arg Lys Val Gln Pro Ser Glu Leu Val
   145                 150                 155                 160
   GGG AGT GTG TGG ACC AAA GAA GAT AAA GAA ATA AAT TCT CCA AAT TTA     528
   Gly Ser Val Trp Thr Lys Glu Asp Lys Glu Ile Asn Ser Pro Asn Leu
                   165                 170                 175
   TTA AAA ATG ATT CGC CAT ACC ACA AAT CTC ACC CTC TGG TTT GAA AAA     576
   Leu Lys Met Ile Arg His Thr Thr Asn Leu Thr Leu Trp Phe Glu Lys
               180                 185                 190
   TGC ATT GTG GAA GCA GAA AAT TTT GAA GAA CGG GTG GCA GTA CTA AGT     624
   Cys Ile Val Glu Ala Glu Asn Phe Glu Glu Arg Val Ala Val Leu Ser
           195                 200                 205
   AGA ATT ATA GAA ATT CTG CAA GTT TTT CGA GAT TTG AAT AAT TTC AAT     672
   Arg Ile Ile Glu Ile Leu Gln Val Phe Arg Asp Leu Asn Asn Phe Asn
       210                 215                 220
   GGC GTA TTG GAG ATA GTC AGT GCA GTA AAT TCA GTG TCA GTA TAC AGA      720
   Gly Val Leu Glu Ile Val Ser Ala Val Asn Ser Val Ser Val Tyr Arg
   225                 230                 235                 240
   CTA GAC CAT ACC TTT GAG GCA CTG CAG GAA AGG AAA AGG AAA ATT TTG      768
   Leu Asp His Thr Phe Glu Ala Leu Gln Glu Arg Lys Arg Lys Ile Leu
                   245                 250                 255
   GAC GAA GCT GTG GAA TTA AGT CAA GAT CAC TTT AAA AAA TAC CTA GTA      816
   Asp Glu Ala Val Glu Leu Ser Gln Asp His Phe Lys Lys Tyr Leu Val
               260                 265                 270
   AAA CTT AAG TCA ATC AAT CCA CCT TGT GTG CCT TTT TTT GGA ATA TAT      864
   Lys Leu Lys Ser Ile Asn Pro Pro Cys Val Pro Phe Phe Gly Ile Tyr
           275                 280                 285
   TTA ACA AAT ATT CTG AAG ACC GAA GAA GGG AAT AAT GAT TTT TTA AAA      912
   Leu Thr Asn Ile Leu Lys Thr Glu Glu Gly Asn Asn Asp Phe Leu Lys
       290                 295                 300
   AAG AAA GGG AAA GAT TTA ATC AAT TTC AGT AAG AGG AGG AAA GTA GCT      960
   Lys Lys Gly Lys Asp Leu Ile Asn Phe Ser Lys Arg Arg Lys Val Ala
   305                 310                 315                 320
   GAA ATT ACT GGA GAA ATT CAG CAG TAT CAG AAT CAG CCG TAC TGC CTA     1008
   Glu Ile Thr Gly Glu Ile Gln Gln Tyr Gln Asn Gln Pro Tyr Cys Leu
                   325                 330                 335
   CGG ATA GAA CCA GAT ATG AGG AGA TTC TTT GAA AAC CTT AAC CCC ATG     1056
   Arg Ile Glu Pro Asp Met Arg Arg Phe Phe Glu Asn Leu Asn Pro Met
               340                 345                 350
   GGA AGT GCA TGT GAA AAA GAG TTT ACA GAT TAT TTG TTC AAC AAA AGT     1104
   Gly Ser Ala Cys Glu Lys Glu Phe Thr Asp Tyr Leu Phe Asn Lys Ser
           355                 360                 365
   TTA GAA ATA GAA CCA CGA AAT TGT AAA CAG CCA CCA CGA TTT CCA CGA     1152
   Leu Glu Ile Glu Pro Arg Asn Cys Lys Gln Pro Pro Arg Phe Pro Arg
       370                 375                 380
   AAA AGT ACA TTT GAA CTA AAA GAA CCA GGA ATA CGA CCA AAT GCA GGA     1200
   Lys Ser Thr Phe Glu Leu Lys Glu Pro Gly Ile Arg Pro Asn Ala Gly
   385                 390                 395                 400
   CGA CAT GGA GAA ACA AGT GGA ACA AGA GGA CAT CCA ACA CCT CTA GAA     1248
   Arg His Gly Glu Thr Ser Gly Thr Arg Gly His Pro Thr Pro Leu Glu
                   405                 410                 415
   AGA GAA CCA TAT AAA ATA GAA TTT GAA AGA ATA GCT GAA ACA GAA CTA     1296
   Arg Glu Pro Tyr Lys Ile Glu Phe Glu Arg Ile Ala Glu Thr Glu Leu
               420                 425                 430
   GAA AGT ACA GTA AGT GCA CCA ACA AGT CCA AAT ACT CCC TCA ACA CCA     1344
   Glu Ser Thr Val Ser Ala Pro Thr Ser Pro Asn Thr Pro Ser Thr Pro
           435                 440                 445
   CCA GTT TCA GCA TCA TCA GAT CAC TCA GTA TTT TTA GAT GTA GAT CTA     1392
   Pro Val Ser Ala Ser Ser Asp His Ser Val Phe Leu Asp Val Asp Leu
       450                 455                 460
   AAC AGT AGT CAC GGA TCA AAC ACA ATC TTT GCA CCT GTG CTA CTA CCA      1440
   Asn Ser Ser His Gly Ser Asn Thr Ile Phe Ala Pro Val Leu Leu Pro
   465                 470                 475                 480
   AAG TCC AAG TCT TTC TTT AGT TCA TGT GGA AGT TTA CAT AAA CTA AGT      1488
   Lys Ser Lys Ser Phe Phe Ser Ser Cys Gly Ser Leu His Lys Leu Ser
                   485                 490                 495
   GAA GAG CCC CTG ATT CCT CCT CCT CTT CCT CCT CGA AAA AAG TTT GAT      1536
   Glu Glu Pro Leu Ile Pro Pro Pro Leu Pro Pro Arg Lys Lys Phe Asp
               500                 505                 510
   CAT GAT GGC TCA AAT TCC AAG GGA AAT ATG AAA TCT GAT GAT GAT CCT      1584
   His Asp Gly Ser Asn Ser Lys Gly Asn Met Lys Ser Asp Asp Asp Pro
           515                 520                 525
   CCT GCT ATT CCA CCG AGA CAG CCT CCT CCT CCA AAG GTA AAA CCC AGA      1632
   Pro Ala Ile Pro Pro Arg Gln Pro Pro Pro Pro Lys Val Lys Pro Arg
       530                 535                 540
   GTT CCT GTT CCT ACT GGT GCA TTT GAT GGG CCT CTG CAT AGT CCA CCT      1680
   Val Pro Val Pro Thr Gly Ala Phe Asp Gly Pro Leu His Ser Pro Pro
   545                 550                 555                 560
   CCG CCA CCA CCA AGA GAT CCT CTT CCT GAT ACC CCT CCA CCA GTT CCC      1728
   Pro Pro Pro Pro Arg Asp Pro Leu Pro Asp Thr Pro Pro Pro Val Pro
                   565                 570                 575
   CTT CGG CCT CCA GAA CAC TTT ATA AAC TGT CCA TTT AAT CTT CAG CCA      1776
   Leu Arg Pro Pro Glu His Phe Ile Asn Cys Pro Phe Asn Leu Gln Pro
               580                 585                 590
   CCT CCA CTG GGG CAT CTT CAC AGA GAT TCA GAC TGG CTC AGA GAC ATT      1824
   Pro Pro Leu Gly His Leu His Arg Asp Ser Asp Trp Leu Arg Asp Ile
           595                 600                 605
   AGT ACG TGT CCA AAT TCG CCA AGC ACT CCT CCT AGC ACA CCC TCT CCA      1872
   Ser Thr Cys Pro Asn Ser Pro Ser Thr Pro Pro Ser Thr Pro Ser Pro
       610                 615                 620
   AGG GTA CCG CGT CGA TGC TAT GTG CTC AGT TCT AGT CAG AAT AAT CTT      1920
   Arg Val Pro Arg Arg Cys Tyr Val Leu Ser Ser Ser Gln Asn Asn Leu
   625                 630                 635                 640
   GCT CAT CCT CCA GCT CCC CCT GTT CCA CCA AGG GAG                      1956
   Ala His Pro Pro Ala Pro Pro Val Pro Pro Arg Glu
                   645                 650
(2) INFORMATION POUR LA SEQ ID NO: 8:
   (i) CARACTERISTIQUES DE LA SEQUENCE:
      (A) LONGUEUR: 652 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 8:
   Arg Phe Glu Ile Pro Glu Pro Glu Pro Thr Glu Ala Asp Lys Leu Ala
   1               5                   10                  15
   Leu Glu Lys Gly Glu Gln Pro Ile Ser Ala Asp Leu Lys Arg Phe Arg
   20 25 30
   Lys Glu Tyr Ile Gln Pro Val Gln Leu Arg Val Leu Asn Val Gln Arg
   35 40 45
   His Trp Val Glu His His Pro His Asp Phe Glu Arg Asp Leu Glu Leu
   50 55 60
   Leu Glu Arg Leu Glu Ser Phe Thr Ser Ser Ala His Arg Ala Lys Ala
   65 70 75 80
   Met Lys Lys Trp Val Glu Ser Ile Ala Lys Thr Ile Arg Arg Lys Lys
   85 90 95
   Gln Ala Gln Ala Asn Gly Val Ser His Asn Ile Thr Phe Glu Ser Pro
   100 105 110
   Pro Pro Pro Ile Glu Trp His Ile Ser Lys Pro Gly Gln Phe Glu Thr
   115 120 125
   Phe Asp Leu Met Thr Leu His Pro Ile Glu Ile Ala Arg Gln Leu Thr
   130 135 140
   Leu Leu Glu Ser Asp Leu Tyr Arg Lys Val Gln Pro Ser Glu Leu Val
   145 150 155 160
   Gly Ser Val Trp Thr Lys Glu Asp Lys Glu Ile Asn Ser Pro Asn Leu
   165 170 175
   Leu Lys Met Ile Arg His Thr Thr Asn Leu Thr Leu Trp Phe Glu Lys
   180 185 190
   Cys Ile Val Glu Ala Glu Asn Phe Glu Glu Arg Val Ala Val Leu Ser
   195 200 205
   Arg Ile Ile Glu Ile Leu Gln Val Phe Arg Asp Leu Asn Asn Phe Asn
   210 215 220
   Gly Val Leu Glu Ile Val Ser Ala Val Asn Ser Val Ser Val Tyr Arg
   225 230 235 240
   Leu Asp His Thr Phe Glu Ala Leu Gln Glu Arg Lys Arg Lys Ile Leu
   245 250 255
   Asp Glu Ala Val Glu Leu Ser Gln Asp His Phe Lys Lys Tyr Leu Val
   260 265 270
   Lys Leu Lys Ser Ile Asn Pro Pro Cys Val Pro Phe Phe Gly Ile Tyr
   275 280 285
   Leu Thr Asn Ile Leu Lys Thr Glu Glu Gly Asn Asn Asp Phe Leu Lys
   290 295 300
   Lys Lys Gly Lys Asp Leu Ile Asn Phe Ser Lys Arg Arg Lys Val Ala
   305 310 315 320
   Glu Ile Thr Gly Glu Ile Gln Gln Tyr Gln Asn Gln Pro Tyr Cys Leu
   325 330 335
   Arg Ile Glu Pro Asp Met Arg Arg Phe Phe Glu Asn Leu Asn Pro Met
   340 345 350
   Gly Ser Ala Cys Glu Lys Glu Phe Thr Asp Tyr Leu Phe Asn Lys Ser
           355                 360                 365
   Leu Glu Ile Glu Pro Arg Asn Cys Lys Gln Pro Pro Arg Phe Pro Arg
       370                 375                 380
   Lys Ser Thr Phe Glu Leu Lys Glu Pro Gly Ile Arg Pro Asn Ala Gly
   385                 390                 395                 400
   Arg His Gly Glu Thr Ser Gly Thr Arg Gly His Pro Thr Pro Leu Glu
                   405                 410                 415
   Arg Glu Pro Tyr Lys Ile Glu Phe Glu Arg Ile Ala Glu Thr Glu Leu
               420                 425                 430
   Glu Ser Thr Val Ser Ala Pro Thr Ser Pro Asn Thr Pro Ser Thr Pro
           435                 440                 445
   Pro Val Ser Ala Ser Ser Asp His Ser Val Phe Leu Asp Val Asp Leu
       450                 455                 460
   Asn Ser Ser His Gly Ser Asn Thr Ile Phe Ala Pro Val Leu Leu Pro
   465                 470                 475                 480
   Lys Ser Lys Ser Phe Phe Ser Ser Cys Gly Ser Leu His Lys Leu Ser
                   485                 490                 495
   Glu Glu Pro Leu Ile Pro Pro Pro Leu Pro Pro Arg Lys Lys Phe Asp
               500                 505                 510
   His Asp Gly Ser Asn Ser Lys Gly Asn Met Lys Ser Asp Asp Asp Pro
           515                 520                 525
   Pro Ala Ile Pro Pro Arg Gln Pro Pro Pro Pro Lys Val Lys Pro Arg
       530                 535                 540
   Val Pro Val Pro Thr Gly Ala Phe Asp Gly Pro Leu His Ser Pro Pro
   545                 550                 555                 560
   Pro Pro Pro Pro Arg Asp Pro Leu Pro Asp Thr Pro Pro Pro Val Pro
                   565                 570                 575
   Leu Arg Pro Pro Glu His Phe Ile Asn Cys Pro Phe Asn Leu Gln Pro
               580                 585                 590
   Pro Pro Leu Gly His Leu His Arg Asp Ser Asp Trp Leu Arg Asp Ile
           595                 600                 605
   Ser Thr Cys Pro Asn Ser Pro Ser Thr Pro Pro Ser Thr Pro Ser Pro
       610                 615                 620
   Arg Val Pro Arg Arg Cys Tyr Val Leu Ser Ser Ser Gln Asn Asn Leu
   625                 630                 635                 640
   Ala His Pro Pro Ala Pro Pro Val Pro Pro Arg Glu
                   645                 650
(2) INFORMATION POUR LA SEQ ID NO: 9:
   (i) CARACTERISTIQUES DE LA SEQUENCE:
      (A) LONGUEUR: 41 bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 9:
      GATATCGAAT TCCGIGTIYT IAAYGTIYTI MGICAYTGGG T 41
(2) INFORMATION POUR LA SEQ ID NO: 10:
   (i) CARACTERISTIQUES DE LA SEQUENCE:
      (A) LONGUEUR: 34 bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 10:
      AAGCTTGAAT TCCKIMKYTT ISWRAARTTI AKIA 34
(2) INFORMATION POUR LA SEQ ID NO: 11:
   (i) CARACTERISTIQUES DE LA SEQUENCE:
      (A) LONGUEUR: 33 bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 11:
      ATCHGTNAGG TACATNCCHA GGTAKGGNAC ACA 33
(2) INFORMATION POUR LA SEQ ID NO: 12:
   (i) CARACTERISTIQUES DE LA SEQUENCE:
      (A) LONGUEUR: 27 bases
      (B) TYPE: acide nucléique
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 12 :
      GCNATHTTYC GNCTNAARAA RACNTGG 27

## Revendications

1. Peptide capable de moduler les niveaux d'échange du GDP sur des complexes p21-GDP comprenant les séquences SEQ ID n° 2, 3, 4, 6 ou 8.

2. Peptide selon la revendication 1 **caractérisé en ce qu'**il ralentit ou inhibe l'échange du GDP sur le complexe p21-GDP.

3. Peptide de séquence SEQ ID n° 2, 3, 4, 6 ou 8.

4. Anticorps ou fragment d'anticorps dirigé contre un peptide selon l'une quelconque des revendications 1 à 3.

5. Anticorps ou fragment d'anticorps selon la revendication 4 **caractérisé en ce qu'**il est dirigé contre la séquence peptidique SEQ ID n° 1.

6. Anticorps ou fragment d'anticorps selon la revendication 5 **caractérisé en ce qu'**il possède la capacité d'inhiber au moins partiellement l'échange du GDP sur le complexe p21-GDP.

7. Séquence nucléotidique codant pour un peptide tel que défini dans l'une quelconque des revendications 1 à 3.

8. Séquence nucléotidique selon la revendication 7 **caractérisée en ce qu'**elle est choisie parmi :
(a) les séquences SEQ ID n° 1, 5 ou 7 et code pour un peptide capable de moduler les niveaux d'échange du GDP sur des complexes p21-GDP,
(b) le brin complémentaire des séquences (a),
(c) les séquences dérivées des séquences (a) et (b) en raison de la dégénérescence du code génétique.

9. Séquence antisens capable d'inhiber la production de peptides selon la revendication 3 et dérivés des séquences SEQ ID N°1, SEQ ID N°5 ou SEQ ID N°7 ou de leur brin complémentaire.

10. Utilisation d'une séquence selon la revendication 8(b) pour la détection *in vitro* de l'expression du facteur d'échange du GDP ou pour la mise en évidence d'anomalies génétiques (mauvais épissage, polymorphisme, mutations ponctuelles).

11. Composition pharmaceutique comprenant comme principe actif au moins un peptide selon l'une des revendications 1 à 3 ou un anticorps ou fragment d'anticorps selon l'une des revendications 4 à 6 ou une séquence nucléotidique selon la revendication 8.

12. Composition pharmaceutique selon la revendication 11 destinée à moduler l'activation des protéines p21.

13. Composition pharmaceutique selon la revendication 12 destinée à inhiber au moins partiellement l'activation des protéines p21.

14. Composition pharmaceutique selon la revendication 11 destinée au traitement des cancers.

15. Utilisation d'un anticorps ou fragment d'anticorps selon l'une des revendications 4 à 6 et/ou d'une séquence nucléotidique selon la revendication 8(b) pour la détection de l'expression et/ou d'une surexpression d'un facteur d'échange du GDP amplifié, muté ou réarrangé dans un échantillon biologique.

16. Utilisation d'un anticorps ou fragment d'anticorps selon l'une des revendications 4 à 6 et/ou d'une séquence nucléotidique selon la revendication 8(b) pour le typage de cancers *in vitro.*

17. Procédé de préparation d'un peptide selon l'une quelconque des revendications 1 à 3 **caractérisé en ce que** l'on cultive une cellule contenant une séquence nucléotidique selon la revendication 7 dans des conditions d'expression de ladite séquence et on récupère le peptide produit.

## Claims

1. Peptide capable of modulating the levels of exchange of GDP with p21-GDP complexes, comprising the sequences SEQ ID No. 2, 3, 4, 6 or 8.

2. Peptide according to Claim 1, **characterized in that** it slows down or inhibits the exchange of GDP with the p21-GDP complex.

3. Peptide of sequence SEQ ID No. 2, 3, 4, 6 or 8.

4. Antibody or antibody fragment directed against a peptide according to any one of Claims 1 to 3.

5. Antibody or antibody fragment according to Claim 4, **characterized in that** it is directed against the peptide sequence SEQ ID No. 1.

6. Antibody or antibody fragment according to Claim 5, **characterized in that** it possesses the capacity to inhibit at least partially the exchange of GDP with the p21-GDP complex.

7. Nucleotide sequence coding for a peptide as is defined in any one of Claims 1 to 3.

8. Nucleotide sequence according to Claim 7, **characterized in that** it is chosen from:
(a) the sequences SEQ ID No. 1, 5 or 7 and that codes for a peptide capable of modulating the levels of exchange of GDP with p21-GDP complexes,
(b) the complementary strand of the sequences (a),
(c) the sequences derived from the sequences (a) and (b) as a result of the degeneracy of the genetic code.

9. Anti-sense sequence capable of inhibiting the production of peptides according to Claim 3 and derived from the sequences SEQ ID No. 1, SEQ ID No. 5 or SEQ ID No. 7 or from their complementary strand.

10. Use of a sequence according to Claim 8(b) for the *in vitro* detection of the expression of GDP exchange factor or for the demonstration of genetic abnormalities (incorrect splicing, polymorphism, point mutations).

11. Pharmaceutical composition comprising as active principle at least one peptide according to one of Claims 1 to 3 or an antibody or antibody fragment according to one of Claims 4 to 6 or a nucleotide sequence according to Claim 8.

12. Pharmaceutical composition according to Claim 11, intended for modulating the activation of p21 proteins.

13. Pharmaceutical composition according to Claim 12, intended for at least partially inhibiting the activation of p21 proteins.

14. Pharmaceutical composition according to Claim 11, intended for the treatment of cancers.

15. Use of an antibody or antibody fragment according to one of Claims 4 to 6 and/or of a nucleotide sequence according to Claim 8(b) for the detection of the expression and/or of an overexpression of an amplified, mutated or rearranged GDP exchange factor in a biological sample.

16. Use of an antibody or antibody fragment according to one of Claims 4 to 6 and/or of a nucleotide sequence according to Claim 8(b) for the *in vitro* typing of cancers.

17. Method for preparing a peptide according to any one of Claims 1 to 3, **characterized in that** a cell containing a nucleotide sequence according to Claim 7 is cultured under conditions for expression of the said sequence, and the peptide produced is recovered.

## Patentansprüche

1. Peptid, das in der Lage ist, die Spiegel des Austausches von GDP an p21-GDP-Komplexen zu modulieren, umfassend die Sequenzen SEQ ID NR. 2, 3, 4, 6 oder 8.

2. Peptid nach Anspruch 1, **dadurch gekennzeichnet, dass** es den Austausch von GDP an dem p21-GDP-Komplex verlangsamt oder hemmt.

3. Peptid der Sequenz SEQ ID NR. 2, 3, 4, 6 oder 8.

4. Antikörper oder Antikörperfragment, der/das gegen ein Peptid nach einem der Ansprüche 1 bis 3 gerichtet ist.

5. Antikörper oder Antikörperfragment nach Anspruch 4, **dadurch gekennzeichnet, dass** er/es gegen die Peptidsequenz SEQ ID NR. 1 gerichtet ist.

6. Antikörper oder Antikörperfragment nach Anspruch 5, **dadurch gekennzeichnet, dass** er/es die Fähigkeit besitzt, den Austausch von GDP am p21-GDP-Komplex zumindest teilweise zu hemmen.

7. Nukleotidsequenz, die für ein Peptid nach einem der Ansprüche 1 bis 3 codiert.

8. Nukleotidsequenz nach Anspruch 7, **dadurch gekennzeichnet, dass** sie aus Folgenden ausgewählt ist:
(a) den Sequenzen SEQ ID NR. 1, 5 oder 7 und für ein Peptid codiert, das in der Lage ist, die Spiegel des Austausches von GDP an p21-GDP-Komplexen zu modulieren,
(b) dem komplementären Strang der Sequenzen (a),
(c) den von den Sequenzen (a) und (b) aufgrund der Degeneration des genetischen Codes abgeleiteten Sequenzen.

9. Antisense-Sequenz, die in der Lage ist, die Produktion von Peptiden nach Anspruch 3 zu hemmen, die von den Sequenzen SEQ ID NR. 1, SEQ ID NR. 5 oder SEQ ID NR. 7 oder ihrem komplementären Strang stammen.

10. Verwendung einer Sequenz nach Anspruch 8 (b) zum In-vitro-Nachweis der Expression des GDP-Austauschfaktors oder zum Nachweis genetischer Anomalien (schlechtes Spleißen, Polymorphismus, Punktmutationen).

11. Pharmazeutische Zusammensetzung, die als Wirkstoff mindestens ein Peptid nach einem der Ansprüche 1 bis 3 oder einen Antikörper oder ein Antikörperfragment nach einem der Ansprüche 4 bis 6 oder eine Nukleotidsequenz nach Anspruch 8 enthält.

12. Pharmazeutische Zusammensetzung nach Anspruch 11, die zur Modulation der Aktivierung von p21-Proteinen bestimmt ist.

13. Pharmazeutische Zusammensetzung nach Anspruch 12, die dazu bestimmt ist, die Aktivierung von p21-Proteinen zumindest teilweise zu hemmen.

14. Pharmazeutische Zusammensetzung nach Anspruch 11, die zur Behandlung von Krebserkrankungen bestimmt ist.

15. Verwendung eines Antikörpers oder Antikörperfragments nach einem der Ansprüche 4 bis 6 und/oder einer Nukleotidsequenz nach Anspruch 8(b) zum Nachweis der Expression und/oder einer Überexpression eines verstärkten, mutierten oder umgeordneten GDP-Austauschfaktors in einer biologischen Probe.

16. Verwendung eines Antikörpers oder Antikörperfragments nach einem der Ansprüche 4 bis 6 und/oder einer Nukleotidsequenz nach Anspruch 8 (b) zur *In-vitro-*Typisierung von Krebserkrankungen.

17. Verfahren zur Herstellung eines Peptids nach einem der Ansprüche 1-3, **dadurch gekennzeichnet, dass** man eine Zelle, die eine Nukleotidsequenz nach Anspruch 7 enthält, unter Bedingungen der Expression dieser Sequenz kultiviert und das produzierte Peptid gewinnt.
